# EUROPEAN PATENT SPECIFICATION

(11) **EP 3 600 454 B1**
(45) Date of publication and mention of the grant of the patent: **07.06.2023**
(21) Application number: 18770802.9
(22) Date of filing: 20.03.2018
(51) Int. Cl.: A61L 2/04, A61L 2/18, A61B 90/70, A61B 1/12, A61B 50/30, F28C 3/00, A61L 2/20, A61L 2/26

(54) **RAPID STERILIZATION IN A DRYING CHAMBER**
SCHNELLE STERILISATION IN EINER TROCKNUNGSKAMMER
STÉRILISATION RAPIDE DANS UNE CHAMBRE DE SÉCHAGE

(30) Priority: 20.03.2017 US 201762473543 P; 13.12.2017 US 201762598004 P
(43) Date of publication of application: 05.02.2020
(73) Proprietor: TekDry International, Inc., Lafayette, CO 80026 (US)
(72) Inventor: COOKSON, Adam R., Broomfield, Colorado 80020 (US); STOREY, Daniel, Longmont, Colorado 80503 (US); THOMAS, Christina K., Eden Prairie, Minnesota 55346 (US)
(74) Representative: D Young & Co LLP
(86) International application number: PCT/US2018/023384
(87) International publication number: WO 2018/175455

(56) References cited:
- EP-A2- 0 898 971
- EP-B1- 0 898 971
- WO-A1-97/15334
- WO-A2-2011/002700
- US-A- 5 160 700
- US-A- 5 508 009
- US-A- 5 667 753
- US-A- 5 792 422
- US-A1- 2013 192 083
- US-B1- 8 689 461

## Description

### BACKGROUND

The proper sterilization of medical devices, surgical instruments, supplies and equipment utilized in direct patient care and surgery is a critical aspect of the modern health care delivery system and directly impacts patient safety.

The Association for the Advancement of Medical Instrumentation (AAMI) defines sterilization as: "A process designed to remove or destroy all viable forms of microbial life, including bacterial spores, to achieve an acceptable sterility assurance level." Sterility is measured by probability expressed as sterility assurance level (SAL). It is generally accepted that a sterility assurance level (SAL) of 10⁻⁶ is appropriate for items intended to come into contact with compromised tissue, which has lost the integrity of natural body barriers. This would include sterile body cavities, tissues and vascular system. A sterility assurance level (SAL) of 10⁻⁶ means that there is less than or equal to one chance in a million that a particular item is contaminated or unsterile following a sterilization process.

Contexts in which medical devices are reused, including where surgical instruments enter normally sterile tissue or the vascular system (e.g., endoscopes, catheters, etc.), typically require sterilization before each use. Improperly sterilized or contaminated medical devices utilized in patient care can contribute to surgical site infection and can pose a serious risk to the patient's safety and welfare, which can result in a serious life threatening infection or even death. Some sterilization processes can be complex and/or involved. The effectiveness of such processes can often involve thorough training of healthcare workers and technicians involved in the reprocessing and sterilization of medical devices, including providing them with continually updated knowledge and understanding of the scientific principles and methods of sterilization utilized in today's health care settings. For example, use of many sterilants and sterilizing equipment can involve health hazards and other inherent risks, which can be greatly minimized through proper education, precautions, policies, etc. Sterilization processes can typically be used wherever patient care is provided and/or wherever infection control is otherwise desired, such as in acute care hospitals, ambulatory surgical centers, outpatient facilities, dental or physician's offices, etc.

Effective sterilization processes, particularly in medical settings, can rely on various conditions. One such condition is ensuring that the sterilization environments is suited to effectively destroy living organisms. For example, some processes rely on the sterilant and sterilizing equipment being validated and appropriate in design and operation to achieve the correct combination of temperature and sterilant combination (and/or other environmental conditions) to be lethal to microorganisms. Another such condition involves thoroughly cleaning the devices to be sterilized to reduce bioburden (e.g., soil). Larger bioburden can frustrate the sterilization process. If bioburden is too great, the established sterilization parameters may not be adequate for effective sterilization. Another such condition involves providing and maintaining intimate and adequate contact between the sterilant and all surfaces and crevices of the device to be sterilized. In practice, different sterilization processes can be used in different contexts (e.g., to yield a different desired SAL), and different processes can require or desire certain conditions.

Typically, sterilization involves use of a physical and/or chemical procedures to destroy microbial life, including highly resistant bacterial pores. Some common sterilizing agents used in healthcare facilities are saturated steam, ethylene oxide gas, hydrogen peroxide gas plasma, liquid chemicals, and ozone. Dry heat can also be used, though dry heat tends to be less common. Among chemical methods of sterilization, the use of hydrogen peroxide and peracetic acid is acquiring considerable prominence due to their bactericidal, sporicidal, and fungicidal properties, which are well known. Peracetic acid is used in aqueous solutions, as a vapor or spray, and is efficient in the sterilization of plastic packaging and the disinfecting of industrial equipment. Such approaches tend to be of particular interest in the food industry, since they tend to leave a food-safe residue consisting of acetic acid, oxygen, water, and hydrogen peroxide.

Various commercial systems are also available for sterilization. One such system is the Contitherm System, which applies hydrogen peroxide in the form of vapor that adheres to the surface of a device being sterilized in the form of a fine film of condensate. The sterilant can be activated with sterile hot air, thereby promoting the elimination of its residue. Another such system is the Freshfill System, which uses the sanitizing agent in the form of a spray. Jets of sterile hot air can activate the peroxide and eliminate the residue. Yet another such system is the Sere System, which uses a mixture of chlorinated water, hydrogen peroxide, and peracetic acid. The device to be sterilized can remain in contact with the sanitizing agent for about 90 seconds, after which, it can be rinsed with sterile water. Another such system is the ethylene oxide (ETO) sterilization system, which tends to be used for sterilizing heat-sensitive materials with a high degree of penetrability of the materials. The ETO system typically heats to 58° C and can also use Freon gas in the process.

Conventional approaches for sterilization (e.g., including conventional sterilization systems that use peracetic acid, hydrogen peroxide, ethylene oxide as well as elevated heat and pressure such as autoclave systems) have a number of limitations. For example, such systems can risk contamination of the materials being sterilized and the sterilization environment during the process. In systems that use sterilization liquids, application of sterilants in diluted form can involve using large volumes of sanitizing liquids, materials can be difficult or impractical to pack, processes may rely on sterile water for rinsing and/or clean areas for sterilizing, etc. Systems that use vapors or sprays can tend to rely on air which is filtered, hot, and sterile to activate and eliminate the residues; and such systems can incur high energy consumption from the use of heater devices. Some processes have further limitations. For example, processes that use ethylene oxide can involve long periods of sterilization, as well as aeration, as the substance can be highly toxic. These and other factors of the various conventional approaches can tend to result in a risk of re-contamination and/or can otherwise frustrate the efficacy of the sterilization. Further, such conventional approaches are often ill suited for devices that include electronic components.

EP 0898971 A2 discloses a multi compartment sterilization system. WO 2011002700 A2 discloses a generation of sterilant gasses and uses thereof.

### BRIEF SUMMARY OF THE INVENTION

Among other things, systems and methods are described for rapid sterilization of devices in a drying chamber. The drying chamber (also referred to as a sterilization chamber or vacuum chamber herein) can create a conductively heated, negative pressure (e.g., vacuum) environment, which can be used to activate and/or otherwise promote the sterilization of devices, including electronic devices, to at least a desired threshold sterilization level (e.g., sterility assurance level, or SAL). In some cases, the devices to be sterilized are medical devices, such as medical implants, instruments, and/or other devices; and the sterilization is to a level acceptable for the medical context of the device. In one embodiment, a medical device (e.g., an endoscope) that has been exposed to excessive contamination is placed inside a sterilization chamber. The sterilizing chamber is closed and a sterilizing routine commences. During the sterilizing routine, the chamber is depressurized to a vacuum level sufficient to gasify liquids inside on device, and the device is conductively heated at least to replace latent heat of vaporization lost during depressurization. The negative pressure and/or heat can cause a sterilant present in the sterilization chamber to be activated. For example, sterilant is provided in the form of a polymeric matrix that will off-gas under vacuum and heat, which can increase the efficacy of the system and can reduce the time for complete sterilization (e.g., to the desired SAL). Further, the off-gassing of the sterilant in a vacuum environment permits expansion (e.g., adiabatic expansion) of the gasified sterilant, which may readily enter into evacuated internal regions of the device.

According to one set of embodiments, a sterilizing system is provided for sterilizing medical instruments. The system includes: a chamber configured to receive a medical instrument; a depressurization subsystem configured, when the medical instrument is in the chamber, to produce a negative pressure environment within the chamber sufficient to gasify liquid and contamination on and in the medical instrument; and a heating subsystem configured to generate heat and comprising a thermal conduction assembly configured, when the medical instrument is in the chamber, to conduct heat to the medical device. In one arrangement, the thermal conductions assembly is further configured to at least partially conform to an external shape of the medical device. In addition, a sterilizing subassembly is provided that off-gasses sterilant in a negative pressure environment. The sterilizing subassembly may include a package of solid-form sterilant within a matrix consisting of, for example, a polymer or other material. The solid-form sterilant contains an encapsulated liquid sterilant with a sufficiently high vapor pressure such that the outgassing, assuming adiabatic expansion of the gas, reaches all surfaces of the product, thus providing a sterile environment. In one arrangement, the sterilizing subassembly further includes a heater (e.g., conductive heater or radiant heater) for heating the solid form-sterilant. In such an arrangement, the medical instrument and the solid-form sterilant may be heated to different temperatures. In another arrangement, the sterilizing subassembly includes a separate chamber that may be depressurized to a negative pressure. In this arrangement, the two chambers may be selectively connected and/or isolated. In one arrangement, the thermal conduction assembly includes a plurality of thermally conductive beads in which the medical instrument may be immersed. In a further arrangement, the thermally conductive beads may be coated with the solid-form sterilant. The medical instrument may include an electrical component that cannot be sterilized via any current sterilization method.

Embodiments relate to a sterilization process, operational devices and respective methods applied for the sterilization of various different items of apparatus and devices, using vacuum techniques and the application of sterilizing gas emitted from a polymer or other matrix. More particularly, some embodiments use gas from a solution of peracetic acid, hydrogen peroxide or other well-known liquid sterilants, encapsulated in a matrix. The matrix consisting of a polymer or other material containing a sterilant with a sufficiently high vapor pressure permits the outgassing of the sterilant in response to vacuum conditions. Further, assuming adiabatic expansion of the gas in vacuum conditions, the off-gassing sterilant may reach all surfaces of the device(s), thus providing a sterile environment.

Various embodiments can include additional features. Some implementations use vibration to cause random movement of the beads and zeolites such that more of the surface area of the coated thermally conductive beads or zeolites to be presented to the vacuum and not shadowed due to intimate contact with other beads or zeolites, thus allowing for more even outgassing from the surface of the beads or zeolites, increasing their lifespan and decreasing the time necessary to sterilize the object. Some implementations introduce ultraviolet light to the inside of the vacuum chamber to provide additional sterilization (e.g., by multimodal sterilization). Some implementations further include use of sterilants that are activated or enhanced by ultraviolet light. Yet other implementations include an ultraviolet-transparent thermally conductive matrix, such as glass or other UV transparent material, treated with a UV transparent polymer matrix that is impregnated with a UV activated sterilant such as photo-activated riboflavin.

In a further implementation, a solid-form sterilant is provided. In various implementations, the solid-form sterilant includes a sterilant matrix and a sterilant compound encapsulated within the sterilant matrix. The solid-form sterilant may include a matrix material selected from the group consisting of a polymer (natural or synthetic), a ceramic, a glass, and combinations thereof. The sterilant compound encapsulated within the sterilant matrix may be in in liquid form. In various implementations, the sterilant compound may be selected from one or more of acids, alcohols, hydrogen peroxide, glutaraldehyde, ortho-phthaladehyde, to name a few. In further implementations, the solid form sterilant may be encased in a gas-permeable container or wrapping.

### BRIEF DESCRIPTION OF THE DRAWINGS

The present disclosure is described in conjunction with the appended figures:
**FIG. 1** shows an embodiment of a sterilizing environment, according to various embodiments;
**FIGS. 2** shows an exemplary medical device;
**FIG. 3A** shows an single chamber embodiment of a sterilizing system;
**FIG. 3B** shows a dual chamber embodiment of a sterilizing system;
**FIG. 4** shows an illustrative computational system for implementing functionality of a sterilizing system, according to various embodiments;
**FIG. 5A** shows a flow diagram of a first illustrative method for sterilizing a medical device, according to various embodiments;
**FIG. 5B** shows a flow diagram of a first illustrative method for sterilizing a medical device, according to various embodiments;
**FIG. 6** shows a graphical representation of an illustrative sterilizing cycle.

In the appended figures, similar components and/or features can have the same reference label. Further, various components of the same type can be distinguished by following the reference label with a second label that distinguishes among the similar components. If only the first reference label is used in the specification, the description is applicable to any one of the similar components having the same first reference label irrespective of the second reference label.

### DETAILED DESCRIPTION

Various conventional sterilizing approaches tend towards harsh environments, which can include steam and pressure, caustic liquids, and radiation. Traditional sterilizing approaches tend to be inapplicable and/or ineffective for sterilizing portable electronic medical devices or other complex items that have a need to be sterile for a number of reasons. One such reason is that current medical devices often include a number of electronic components (e.g., processors, batteries, etc.) housed in a substantially sealed environment. The housing can frustrate attempts to remove the contamination (e.g., by limiting contact between internal components and absorptive materials) and can slow "normal" sterilizing of the internal component (e.g., from ambient air). Still, it can be undesirable to open up the device to expose the internal components for sterilizing, as that can cause further damage, break seals, void warranties, etc.

Another such reason is that, as liquid and contamination leaves the device (e.g., by evaporation), it can remove latent heat from the device, which can effectively freeze remaining liquid. This can further frustrate attempts to remove the remaining liquid or contamination from the device. Yet another such reason is that the devices are often made of materials that are sensitive to scratching, over-exposure to pressure and/or temperature, etc. For example, plastics or metals of different colors or finishes can respond differently to different temperatures, pressures, and/or contact with other materials. Similarly, a display screen can be easily scratched or cracked if exposed to certain environments. Accordingly, approaches that involve contact with certain types of materials, radiant heating, high pressures, high temperatures and/or other types of exposure can cause damage to the device being sterilized. Similarly, the devices often include various ports, sensors, and/or other components that have their own sensitivities. For example, typical sterilants such at hydrogen peroxide or peracetic acid are very caustic above a certain concentration. This can cause damage to the sensitive electronics or discoloration or damage to medical devices. These and other considerations can constrain the types of sterilizing techniques that can be used with these devices and can limit effectiveness of those techniques.

Embodiments provide novel systems and methods for sterilizing an medical device having electrical components (i.e., electrical medical device), non-electronic medical device, or instrument by conductively heating the device with a conductive heating assembly within a depressurized chamber (e.g., vacuum chamber) that includes an evaporable sterilant. The chamber can apply negative pressure to cause any liquid on or in the device to gasify and leave the device, destroying any bacteria, while the conductive heating assembly supplies heat to the device. In some implementations, the heat supplied by the conductive heating assembly can be enough to avoid freezing during removal of liquid from the device. In other implementations, additional heat is applied to the device to further aid the sterilizing Some embodiments of the conductive heating assembly are designed to gently and evenly supply conductive heat to the device without damaging the device, for example, through scratching, overheating, etc. Additionally, the evaporable sterilant used can be delivered locally, thus avoiding the issue of corrosion or other issues these sterilants can cause in higher concentrations.

Some embodiments involve a pressure that is lower than the vapor pressure of the sterilant per the ideal gas law of pV=nRT, where p is the pressure in the chamber, V is the volume of the chamber, R is the ideal gas constant (0.082057 L atm mol⁻¹K⁻¹), n is the number of moles of the gas, and T is the temperature of system. From this equation, with a constant volume and amount of gas, the amount of pressure needed to evaporate the sterilant increases with an increase of the temperature of the system in a linear fashion.

In the following description, numerous specific details are set forth to provide a thorough understanding of various embodiments. In illustrated embodiments, the conductive heating assembly is configured to conform to the device within the chamber to better supply heat to the device and prevent the potential of freezing. However, it will be appreciated that the present disclosure is not limited to conformable conductive heating assemblies. Further, references to beads or zeolites or other types of conformal materials herein are intended to include any suitable media that may or may not be coated with one or more type of sterilant (e.g., liquid sterilant and/or one or more type of polymer matrix). However, one having ordinary skill in the art should recognize that the invention can be practiced without these specific details. In some instances, circuits, structures, and techniques have not been shown in detail to avoid obscuring the present invention.

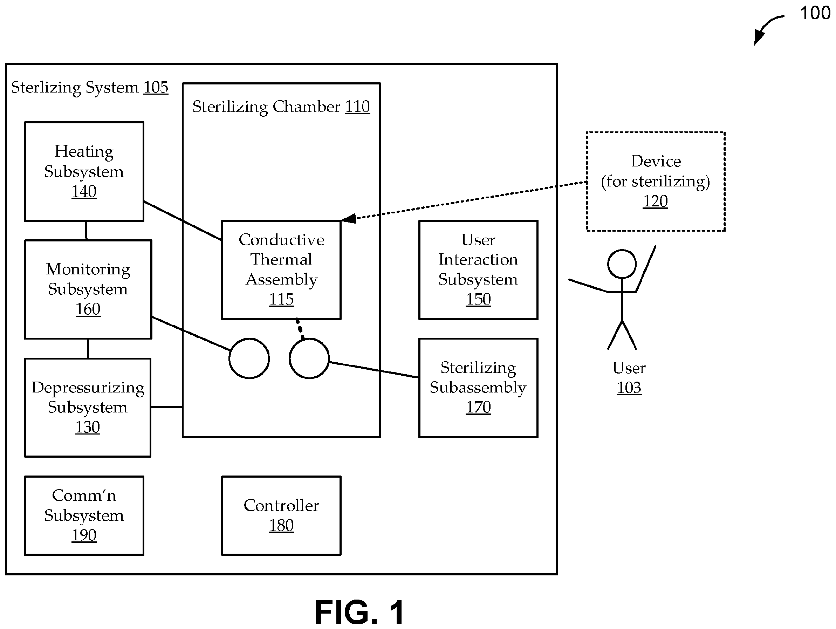

Turning first to FIG. 1, a block diagram is shown of an embodiment of a sterilizing environment 100, according to various embodiments. The sterilizing environment 100 includes a sterilizing system 105 that can be used by users 103 to dry and/or sterilize any one or more suitable device(s) 120. The device(s) 120 can be electrical medical devices, non-electronic medical devices, instruments, and other medical or non-medical items. For example, the sterilizing system 105 can be used to sterilize devices 120 that have been overexposed to microbial contaminants (e.g., including liquid contamination), cleaning solutions, etc. The device(s) 120 can be placed into a sterilizing chamber 110 where contact is established with a conductive thermal assembly 115 and sterilizing subassembly 170. As described herein, in some implementations, the sterilizing subassembly 170 is a separate assembly from the sterilizing chamber. In other implementations, the sterilizing assembly 170 is positioned within the sterilizing chamber. In yet further implementations, the sterilizing assembly is part of the conductive thermal assembly 115 (e.g., as a sterilant coating on thermally conductive beads/conformable media). Negative pressure (e.g., a partial vacuum) is applied to the sterilizing chamber 110 by a depressurizing subsystem 130, and heat is applied to the device(s) 120 via the conductive thermal assembly 115 using a heating subsystem 140. In further implementations, the heating subsystem 140 or a separate heating subsystem may apply heat to the sterilizing subassembly.

The sterilizing subassembly 170 can include any suitable sterilant and delivery mechanism. For example, implementations of the sterilizing subassembly 170 can include sterilant packages disposed within the chamber, sterilant packages disposed within a separate chamber in fluid communication with the chamber, coated beads, zeolites, ultraviolet radiation sources, vibration, and/or other elements that can be activated in response to low pressure conditions and activated with sufficient intensity (e.g., concentration, amount, etc.) and/or amount of time to ensure sterilization to at least a predetermined sterilization level (e.g., a SAL of 10⁻⁶). In some implementations, the sterilizing subassembly 170 can include a liquid sterilant encased in a polymer or other matrix that can be released (e.g., off-gassed) upon heating, low pressure, or a combination of the two. Such a liquid sterilant disposed within a matrix is sometimes referred to, herein, as a solid-form sterilant. In other implementations, the sterilizing subassembly 170 can include liquid sterilant encased in a matrix that coats thermal conductive beads or zeolites that form part of the conductive thermal assembly. An illustrative, non-exhaustive list of sterilants is shown in TABLE 1:

| **Manufacturer** | **Active ingredient(s)** |
|---|---|
| **K091210 Acecide-C High Level Disinfectant and Sterilant** | |
| Best Sanitizers, Inc. | 3100-3400 ppm peracetic acid |
| **K091890 Extended Use Aldahol High Level Disinfectant** | 20.1% isopropanol |
| Healthpoint, Ltd. | |
| **K091022 ResertXL HLD High Level Disinfectant** | |
| Steris Corporation | 2.0% hydrogen peroxide |
| **K090036 Steris S40 Sterilant Concentrate** | 2.0% hydrogen peroxide |
| Steris Corporation | |
| **K090036 Steris S40 Sterilant Concentrate** | 2.0% hydrogen peroxide |
| Steris Corporation | ≥1820 mg/L peracetic acid |
| **K113015 Aldahol V High Level Disinfectant** | ≥1820 mg/L peracetic acid |
| Microchem Laboratories, Inc. | 3.4% glutaraldehyde |
| **K120306 Rapicide OPA-28 High Level Disinfectant** | |
| Minntech Corporation | 0.575% *ortho*-phtnalaldehyde |
| **K1140703 OPA 30 Solution** | |
| (iden Technologies, LLC | 0.6% *ortho*-phthalaldehyde |
| **K041984 Acecide High Level Disnfectant and Sterilant** | |
| Best Sanitizers, Inc. | 3300-3800 ppm peracetic acid |
| **K051305 TD-5 High-level Disinfectant** | |
| CS Medical, L.L.C. | 2.65% glutaraldehyde |
| **K0431360 Aldahol III High Level Disinfectant** | 3.4% glutaraldehyde |
| Healthpoint LTD | 26% isopropanol |
| **K013280 Sterilox Liquid High Level Disinfectant System** | Hypochlorite and |
| | Hypochlorous acid 650-675 ppm Active free chlorine |
| Sterilox, Technologies, Inc. | |
| **K003087 Sporicidin Sterilizing and Disinfecting Solution** | 1.12% glutaraldehyde |
| | 1.93% phenol/phenate |
| Sporicidin International | |

A more complete listing of possible sterilant is provides in U.S. Provisional Application No. 62/473,543, which is incorporated by reference above.

By way of example, hydrogen peroxide, peracetic acid, or other sterilants can be suitable for inclusion within a matrix that can be released upon a threshold level of heating and/or negative pressure. An illustrative, non-exhaustive list of matrices that can be used with such sterilants include, for example, Polyphosphazenes, Poly anhydrides, Polyacetals, Poly(ortho esters), Polyphosphoesters, Polycaprolactone, Polyurethanes, Polylactide, Polycarbonates and/or Polyamides. By way of further example, the matrix can include polyethylene glycol (PEG), zeolites, or other suitable substances. The sterilizing subassembly may further include one or more heaters to heat the solid-form sterilant.

In some embodiments, a monitoring system 160 tracks the sterilization process. For example, the monitoring system 160 can record and log a set of parameters for use in monitoring compliance with a quality process or standard, such as the ISO 13485 quality standards. In some implementations, the set of parameters and/or other information can be fed back (e.g., to the controller 180) to adjust the environment of the sterilization chamber 110.

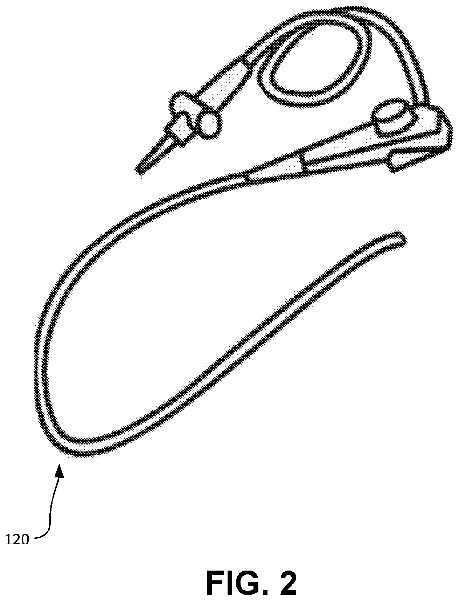

The sterilizing environment 100 can be used to treat any suitable type of device 120 to be sterilized. For example, in a medical context, the device 120 can be an electromechanical device (e.g., insulin pump, injector, etc.), portable computer monitoring system (e.g., tablet, laptop, etc.), surgical implement (e.g., scalpel, trocar, etc.), cauterizer, portable audio and/or video recording device (e.g., voice recorder, camera, video recorder, etc.), portable imaging device, implantable device (e.g., orthopedic implant, etc.), etc. As shown in FIG. 2, one non-limiting exemplary medical device is an endoscope 120. As will be appreciated, many such endoscopic devices include various electronic components (e.g., electrodes, cameras etc.), which make sterilizing these devices problematic. Further, the illustrated medical device includes various internal lumens, which have previously frustrated sterilization efforts. Typically, the device 120 has exposure limits (e.g., set by the manufacturer) for one or more environmental conditions, such as temperature, exposure to caustic sterilants, radiation or heat. For example, many devices 120 can have relatively low exposure limits for temperature, caustic environments, liquids, radiation, etc. Accordingly, embodiments can use negative pressure (e.g., vacuum) to facilitate a "cool" flash boiling of liquid inside the device 120; and a controlled, relatively low temperature can be used to facilitate the sterilizing, while remaining well within the thermal exposure limits of the device. At the same time, due to the drop-in pressure from the negative pressure/vacuum, it is possible to release encapsulated sterilant (e.g., liquid sterilant disposed within a matrix) locally, thus providing sufficient sterilant to sterilize to a desired level without overexposing the device 120 to sterilant, which can cause damage.

Embodiments of the sterilizing chamber 110 are manufactured in any suitable manner in any suitable size and of any suitable shape and material, so that desired number and/or types of devices 120 can fit within the chamber, and the chamber can support the types of negative pressure applied to it by the depressurizing subsystem 130. For example, the sterilizing chamber 110 can be made of metal or sturdy plastic and can include seals, where appropriate, to maintain appropriate levels of negative pressure within the sterilizing chamber 110. Some implementations include multiple sterilizing chambers 110 for concurrent, but segregated sterilizing of multiple devices 120, or for sterilizing of different sizes and/or shapes of devices 12- (e.g., with correspondingly sized and/or shaped sterilizing chambers 110). Some are designed to facilitate use within context of a larger assembly (e.g., a wall-mounted or case-integrated sterilizing chamber 110). In one implementation, multiple sterilizing chambers 110 are stacked in a configuration that allows access like a drawer, chest, etc.). Some implementations further include windows, internal lighting (such as UV light), and/or other features to allow users 103 to view the inside environment (e.g., during sterilizing of their device(s) 120). In such an implementation, the beads may be substantially transparent to permit viewing a medical device and/or UV light to reach the medical device.

The sterilizing chamber 110 is pressurized by a depressurizing subsystem 130. Embodiments of the depressurizing subsystem 130 include a vacuum pump or the like for producing a negative pressure environment within the sterilizing chamber 110. The specifications of the depressurizing subsystem 130 are selected to produce a desired vacuum level within a desired amount of time, given the air-space within the sterilizing chamber 110, the quality of the sterilizing chamber 110 seals, etc. In one embodiment, the depressurizing subsystem 130 includes a one-half-horsepower, two-stage vacuum pump configured to produce a vacuum level within the sterilizing chamber 110 of less than 100 Torr, more preferably less than 10 Torr and yet more preferably less than 1 Torr and to maintain substantially that level of vacuum throughout the sterilizing routine (e.g., for fifteen to thirty minutes). Different depressurizing subsystem 130 specifications can be used to support concurrent sterilizing in multiple sterilizing chambers 110, sterilizing in sterilizing chamber 110 of different sizes, use in portable versus hard-mounted implementations, etc.

In some embodiments, the depressurizing subsystem 130 is in fluid communication with the sterilizing chamber 110 (or multiple sterilizing chambers 110) via one or more fluid paths. For example, a fluid path can include one or more release valves, hoses, fittings, seals, etc. The fluid path components are selected to operate within the produced level of negative pressure. Certain embodiments include an electronically controlled (or manual in some implementations) release valve for releasing the negative pressure environment to allow the sterilizing chamber 110 to be opened after the sterilizing routine has completed (or at any other desirable time). This release valve has the ability to contain a filter such that room air can be allowed in the chamber without causing recontamination.

In another embodiment, the release valve is attached to a container of sterilized, pressurized gas such as pure argon or pure nitrogen. In implementations including multiple sterilizing chambers 110, multiple fluid paths, multiple release valves, or other techniques can be used to fluidly couple the pressurizing subsystem 130 with the sterilizing chambers 110.

Depressurization of the sterilizing chamber 110 by the depressurizing subsystem 130 causes liquid on and in the device(s) 120 to gasify (e.g., evaporate, vaporize, etc.). It can also engender the release of sterilant from the sterilizing subassembly 170. For example, liquid inside the device(s) 120 can become vaporized and can escape from various ports and other non-sealed portions of the housing. The sterilant from the sterilizing subassembly 170 can follow the same path into the device, thus flooding the device with a desired amount of sterilant. Evaporation of the liquid away from the device(s) 120 is an endothermic process (i.e., involving latent heat) that causes a temperature drop in the sterilizing chamber 110 around the device(s) 120. In some implementations, this can frustrate (e.g., slow) the sterilizing process. Accordingly, some embodiments add heat to the sterilizing chamber 110. In some implementations, the amount of heat added to the environment is only as much as sufficient to overcome the latent heat of vaporization. In other implementations, other amounts of heat are provided to the environment within the sterilizing chamber 110. For example, additional heat can be added to activate and/or speed up the sterilizing process, or heat can be added in varying amounts over time for various purposes. For example, the amount of heat (e.g., and/or a profile of changes in temperature and/or pressure over time) can be tailored to particular implementations of encapsulated sterilants and corresponding vapor pressures for release of those sterilants.

Some conventional approaches to sterilizing involve convective or radiated heat. Convective heating tends not to be useful in context of a negative pressure environment, as the substantial vacuum may not leave sufficient gas molecules in the sterilizing chamber 110 to provide efficient or effective heat transport. Radiated heat can be effective in a vacuum, but can be undesirable for use with many electronic and/or other devices 120 that may be sensitive to certain types and/or levels of radiation. Experimentation by the inventors has demonstrated that typical limits on many devices 120 can often limit the amount of radiated heat and/or sterilant that can be applied to the device(s) 120 to a level that is too low to be effective and/or can cause the device(s) 120 to absorb too much heat in one region and not enough in another, to be sterilized to an incomplete level, etc.

Embodiments described herein use conductive heat to provide heating to the device(s) 120 within the sterilizing chamber 110. A heating subsystem 140 heats a conductive thermal assembly 115 (e.g., and the sterilizing subassembly 170 in some implementations), which is in contact with the device(s) 120 and is configured to conduct heat to the device(s) 120. Generally, the conductive thermal assembly supports and heats a device within the chamber. Implementations of the conductive thermal assembly 115 at least partially conform to an external shape of the device(s) 120 so as to at least partially surround the portable electronic medical device 120. For example, the conductive thermal assembly 115 can be designed so that the portable electronic medical device, non-electronic medical device, instrument or other device 120 is gently immersed in, sandwiched between, or otherwise in conformed contact with elements of the conductive thermal assembly 115. For example, conductive beads or zeolites, heat packs, etc., can be assembled in a manner that dynamically conform to the geometry of one or more types of portable electronic medical devices, non-electronic medical devices, etc. when such device(s) 120 are moved into contact with the conductive thermal assembly 115. Other embodiments of the conductive thermal assembly 115 statically conform to the shape of the portable electronic medical device 120. In one such embodiment, a custom partial or total encasement is designed and manufactured to fit one or more particular devices 120 shape. For example, a custom-manufactured conductive plate is designed as a conductive thermal assembly 115 (e.g., and sterilizing subassembly 170) that interfaces between the conveyor assembly 125 and a particular type (e.g., make and/or model) of device 120. Examples of various conformable conductive thermal assemblies are set forth in co-owned U.S. Patent No. 8,689,461, the entire contents of which are incorporated herein by reference.

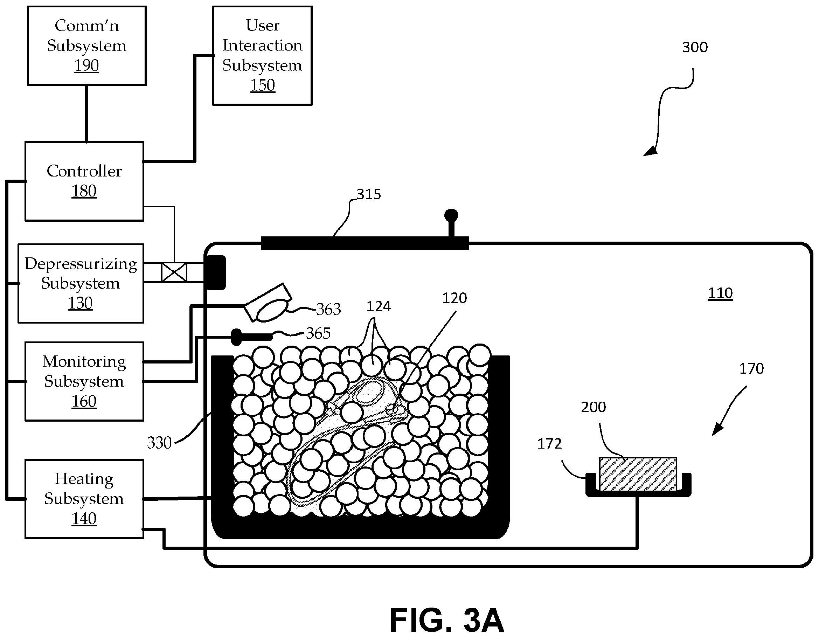

As shown in FIG. 3A, one implementation of the conductive thermal assembly 115 includes a number of thermally conductive beads 124. For example, a portion of the sterilizing chamber 110 is partially filled with small aluminum spheres or other conductive beads (e.g., zeolites), which need not be spheres, sized to be small enough to substantially conform to the shape of the device(s) 120 when the device(s) 120 are placed in the beads (e.g., partially or fully submerged into the bed of beads). Of note, the beads 124 and/or the medical device are not to scale. The spheres or beads (hereafter beads) 124 are also sized to be larger than any port or opening in the device(s) 120. In such an implementation, the heating subsystem 140 can heat the sterilizing chamber 110 from the outside (e.g., from the bottom and/or sides of the sterilizing chamber 110). The applied heat (e.g., thermal energy) from the heating subsystem 140 (e.g., resistive electrical heater or radiant heater that heats the beads) is conducted toward the device(s) 120 via the beads, permitting the heat to evenly and gently surround at least a portion of the device(s) 120. Experimentation by the inventors has demonstrated that the beads tend to store heat in their mass, so that cooling from the latent heat of vaporization can be counteracted by heat stored in the beads adjacent to the device(s) 120. Some implementations select beads having relatively high thermal capacity (e.g., storage), which can tend to provide a steady flow of heat to the device(s) 120 without exceeding maximum temperature limits. For example, beads with low thermal conductivity and/or low heat storage capacity can tend to allow cold regions to form around the device(s) 120 as the liquid gasifies, potentially quenching the gasification of the liquid once the temperature drops below a phase change temperature at that level of vacuum. In context of those low thermal conductivity and/or low heat storage capacity beads, further increases in heat could have limited impact due to the low thermal conductivity of the beads, and can potentially conduct a "slow wave" of too much heat and cause damage to the device(s) 120.

For the sake of illustration, a non-limiting set of various potential materials are analyzed for use as beads in TABLE 2:

| Material | Specific Heat | Conductivity | Softness (10 - hardness) | Score (Factor Product) |
|---|---|---|---|---|
| | | | | |
| Magnesium | 1.05 | 156 | 8 | 1310.4 |
| Aluminum | 0.87 | 205 | 7.1 | 1266.3 |
| Copper | 0.39 | 401 | 7 | 1094.7 |
| Sodium | 1.26 | 84 | 9.6 | 1016.1 |
| Gold | 0.13 | 310 | 7 | 282.1 |
| Brass | 0.38 | 109 | 6 | 248.5 |
| Cadmium | 0.25 | 92 | 8 | 184.0 |
| Indium | 0.13 | 147 | 3.5 | 66.9 |
| Antimony | 0.21 | 18.5 | 6.7 | 26.0 |
| Asphalt | 0.92 | 0.75 | 8 | 5.5 |
| Glass | 0.84 | 1.05 | 3.5 | 3.1 |
| Mica | 0.50 | 0.71 | 7.2 | 2.6 |
| Zeolite | 0.012 - 1.3* | 1 - 100* | 1-9* | 0.012 - 1,170* |

| | | | | |
|---|---|---|---|---|
| * depends on zeolite material. | | | | |

TABLE 2 evaluates three criteria of different materials: specific heat, conductivity, and softness. The specific heat and conductivity indicate the material's ability to store and conduct heat, and the softness indicates whether the material is likely to damage portions of the sterilizing environment 100 (e.g., surface coatings, displays, etc.). A score is calculated as a function of the three criteria values (e.g., and/or other values). The illustrated score is computed as a product of the values without any weighting. Other implementations compute the score by a weighted product of the values. In other implementations, additional or alternate factors can be used as part of the score computations, such as ease of manufacturing, access, cost, susceptibility to contamination, ease of cleaning, ability to hold and maintain a coating matrix with a liquid sterilant, etc. In an illustrative implementation based on the above table, while magnesium achieved a slightly better score than aluminum, aluminum was chosen as a more cost-effective option. In some embodiments, custom alloys, composites, and/or other materials are used to achieve better scores, according to the above and/or different criteria.

Bead geometry can also be selected based on various considerations. One such consideration is the opening size of the electronic medical device, non-electronic medical devices, instruments and other medical items 120. It is desirable to keep beads from entering any ports, sockets, or other openings in the electronic medical device, non-electronic medical devices, instruments and other medical items (i.e., medical devices 120). For example, an endoscope having a 3.5 millimeter lumen opening, and the next-largest available common size for aluminum balls is chosen (e.g., six-millimeter BBs). Another such consideration is that increasing contact surfaces can increase heat conduction. For example, dodecahedron and other regular polyhedrons may provide more conduction than a sphere or other shape and can be selected accordingly. The shape can be selected to provide more conduction surface with the device(s) 120 and/or with the heat source (e.g., walls of the sterilizing chamber 110). Still another such consideration is to shape the beads to permit conformance with the exterior geometry of the device(s) 120. Conduction to a flat surface or very large beads or zeolites on which the device is placed may only heat some or all of a single surface of the device, while smaller beads can allow the device to be partially or fully immersed within the conductive elements.

In some implementations, the device(s) 120 are placed in a heat-conductive shield before being placed in the sterilizing chamber 110 with the beads. The heat conductive shield such as a porous bag (e.g., mesh bag) or semi-porous bag can protect the surface of the device(s) 120 from scratches or other damage caused by contact with the beads, and/or can further distribute the heat from the beads. For example, a medical device can be encased in a paper or mesh wrap prior to submerging the device in the beads. The heat-conductive shield can also facilitate wicking of moisture away from the device(s) 120. For example, the paper wicks moisture away from the device as it dries, which can mitigate formation of stains or "water spots" on the surface of the device as the liquid escapes the device and evaporates. In any implementation, the shield should have be porous to permit gasified sterilant to access the encased device. That is, the shield does not affect the sterilization process. Some implementations use specially designed beads (e.g., zeolites) that aid with moisture wicking and/or absorption.

Various embodiments can include different numbers, materials, shapes, sizes, etc. of beads. Some implementations include a conveyor assembly (not shown) configured to hold the device(s) 120 in place within the conductive thermal assembly 115. For example, the conveyor assembly can include a tray, clips, frame, etc. for supporting the device(s) 120. Alternatively, the conveyor assembly can be features of the sterilizing chamber 110, for example, protrusions from the wall or floor of the sterilizing chamber 110. Some implementations of the conveyor assembly move the device(s) 120 into (and/or out of) place within the sterilizing chamber 110 as appropriate. For example, the device(s) 120 can be placed on the conveyor assembly when the sterilizing chamber 110 is open, and closing the sterilizing chamber 110 can cause the conveyor assembly to move the device(s) 120 into contact with the conductive thermal assembly 115. U.S. Patent No. 8,689,461, as incorporated above, illustrates such a conveyor assembly. While the above embodiments are described with reference to the use of heated beads as a conductive thermal assembly, it will be expressly understood that, many other types of conductive thermal assembly 115 are possible including conformable and non-conformable assemblies.

Returning to FIG. 1, other subsystems are used in some embodiments to provide additional functionality. Some embodiments include a monitoring subsystem 160 that can provide feedback control, environmental monitoring within the sterilizing chamber 110, monitoring of the device(s) 120, etc. Implementations of the monitoring subsystem 160 include one or more probes, sensors, cameras, and/or any other suitable device. In one embodiment, the monitoring subsystem 160 includes one or more sensors situated inside the sterilizing chamber 110 and configured to monitor internal pressure (vacuum level), humidity, temperature, and sterilization level within the sterilizing chamber 110. For example, the measurements can be used to determine if the heating is sufficient to overcome the latent heat of vaporization, to determine if the vacuum level is sufficient, to determine when the device(s) 120 has dried sufficiently, to determine if enough sterilant has been released to completely sterilize the component, etc.

The monitoring subsystem 160 can communicate its measurements through wired and/or wireless communications links to a controller 180 located outside the sterilizing chamber 110. For example, the controller 180 includes memory (e.g., non-transient, computer-readable memory) and a processor (e.g., implemented as one or more physical processors, one or more processor cores, etc.). The memory has instructions stored thereon, which, when executed, cause the processor to perform various functions. The functions can be informed by (e.g., directed by, modified according to, etc.) feedback from the monitoring subsystem 160. For example, the measurements from the monitoring subsystem 160 can be used to determine when to end the sterilizing routine and release a pressure release valve of the sterilizing chamber 110, when and how to modify the heat being delivered to the conductive thermal assembly 115, etc. The controller 180 can also direct operation of other subsystems, such as the conveyor assembly 125, pressurizing subsystem 130, etc.

In some embodiments, the monitoring subsystem 160 includes a camera configured to "watch" the internal environment of the sterilizing chamber 110. In one implementation, the camera is used to monitor the vaporization of liquid from the device(s) 120. In another implementation, the camera uses infrared to indicate internal temperature readings from within the sterilizing chamber 110 and/or around the surface of the device(s) 120. In yet another implementation, the camera can monitor functionality of the device(s) 120 within the sterilizing chamber 110. For example, device(s) 120 may be plugged in within the sterilizing chamber 110, and a signal can be sent to the device(s) 120 (e.g., a monitoring message can be sent to the device(s) 120) within the sterilizing chamber 110 to see if the device(s) 120 react. The camera can be used to visually monitor the reaction to determine whether the device(s) 120 was unharmed. In some implementations, the camera is used for other functions, for example, to capture "before" imagery of the device(s) 120 to help determine whether the device(s) 120 had pre-existing conditions (e.g., a cracks etc.) prior to using the sterilizing system 105.

In support of that and/or other functionality, some embodiments of the monitoring subsystem 160 include one or more interface cables. The interface cable can connect the portable electronic medical device, instruments and other device(s) 120 to a power source, a storage device, a communications network, a remote interface, etc. to allow operation of the portable electronic medical device, instruments and other device(s) 120 to be monitored, verified, or even exploited. For example, the interface cable can be used to charge the device(s) 120 during the sterilizing routine or to provide power when the battery is removed. In some implementations, the functionality of the device(s) 120 re verified at the end of the sterilizing routine to determine appropriate next steps. For example, when it is determined that the device(s) 120 has been damaged (i.e., it is non-functional after sterilizing), the monitoring subsystem 160 can provide the user 103 with a warning. In some embodiments, the interface cable is used to extract information from the device(s) 120. For example, an identification number, network provider, tablet number, email address, user identity, and/or other information can be extracted for tracking and/or other purposes.

Some embodiments of the system further include a disinfecting subsystem (not shown) for disinfecting the device(s) 120 prior to sterilization. In one such embodiment, the disinfecting subsystem includes an ultraviolet lamp, or the like. It is common for the surfaces of devices 120 to be rife with bacteria, pathogens, and other contaminants. The lamp can irradiate the internal environment of the sterilizing chamber 110 to help kill many of the contaminants. Typically, use of an ultraviolet lamp alone does not result in a sterilization level considered "sterile" for medical and/or other applications (e.g., SAL of 10⁻⁶). Still, use of ultraviolet radiation can help speed the sterilization process, augment the sterilization process, be a substitute for the sterilization process in some cases, help sterilize the chamber between uses, etc.

In other implementations, the disinfecting subsystem (not shown) can include nozzles and/or other components used to spray or otherwise distribute disinfectants (e.g., solutions of alcohol, bleach, etc.) into the sterilizing chamber 110 in the presence and/or absence of the device(s) 120. Some embodiments include a reservoir or repository for components of the disinfecting subassembly that can be separate from the sterilizing chamber 110. The disinfecting subsystem can be configured to disinfect those additional reservoirs, repositories, etc. in the presence or absence of the conductive thermal assembly 115. In one implementation, the sterilizing chamber 110 partially fills with a disinfecting solution (e.g., an alcohol solution) to bathe the device(s) 120 during (e.g., at the start of) the sterilizing process. The solution is evacuated from the chamber and is quickly boiled off of, and out of, the device(s) 120 during the sterilizing routine (e.g., the solution can also be formulated to facilitate faster evaporation, to help draw other liquids from the device(s) 120, etc.).

Some embodiments of the sterilizing system 105 further include a user interaction subsystem 150 that facilitates user 103 interaction with functions of the system (e.g., using one or more displays, interface devices, quality interfaces, etc.). In some implementations, functionality of the user interaction subsystem 150 is facilitated by the controller 180. In other implementations, the user interaction subsystem 150 is a dedicated system in communication with the controller 180. For example, the user interaction subsystem 150 can be implemented as a tablet computer or other self-contained system with at least one interface (e.g., wired or wireless) between it and other components and subsystems of the sterilizing system 105 (e.g., the controller 180). Embodiments can also perform other functions by exploiting communications functionality through a communications subsystem 190. For example, certain functionality can be performed via the "cloud" or any suitable public or private network, as described more fully below.

Embodiments of the user interaction subsystem 150 can be designed to perform many different types of functions, depending, for example, on the particular implementation of the sterilizing system 105. For example, different models can support different functions, and different models can be tailored for implementation in a business establishment (e.g., a form factor similar to an automated teller machine (ATM) or automated external defibrillator (AED)), as a portable sterilizing system in a case (e.g., a briefcase or toolbox form factor), or as a stand-alone systems for a medical facility, etc.

Referring again to FIG. 3A, an embodiment of a sterilizing system 300, which may be utilized in a medical facility, is shown.. The sterilizing system 300 can be a non-limiting embodiment of sterilizing system 105 of FIG. 1, and its components are described using the same reference numbers, where appropriate, for the sake of added clarity. The sterilizing system 300 is designed to receive device(s) 120 into the sterilizing chamber 110 via a door 315. For example, the door 315 may be disposed on a top surface of the chamber 110 and includes any gaskets or other seals to allow the sterilizing chamber 110 to be sufficiently sealed when the door 315 is closed and the sterilizing chamber 110 is pressurized. A similar form factor can be designed to support multiple sterilizing chambers 110 for concurrent sterilizing (and/or disinfecting) of multiple device(s) 120 and/or for sterilizing of multiple types of device(s) 120.

The sterilizing chamber 110 is depressurized by a depressurizing subsystem 130 (e.g., a vacuum pump or the like in fluid communication with the sterilizing chamber 110 via suitable hoses, seals, valves, etc.). A heating subsystem 140 is coupled with the sterilizing chamber 110 in such a way as to provide heat to a conductive thermal assembly 115 and sterilizing subassembly 170 inside the sterilizing chamber 110. As illustrated, the conductive thermal assembly 115 can include a number of thermally conductive beads 124 supported on or within a first resistive heating element 320. However, other thermal conductive assemblies are possible (e.g., heated plates, etc.). Operation of the heating element 320 heats the thermally conductive beads. In one arrangement, the beads 124 may be coated with a solid-form sterilant. In such an arrangement, the beads define the sterilizing subassembly. In the illustrated embodiment, the sterilizing subassembly is formed of a separate package 200 of solid-form sterilant that may be disposed within the chamber 110. The sterilizing subassembly may further include a second heater 172 for separately heating the package 200 of sterilant. In the illustrated embodiment, the second heater 172 is a second resistive heating element 172 that may support and conductively heat the sterilant package 200. The second heater 172 is controlled by the heating subsystem 140. It will be appreciated that the second heater may take alternate forms (e.g., a radiant heater focused on the package, etc.). In any embodiment, the medical device 120 and the sterilant package 200 may be heated to different temperatures. The sterilizing chamber 110 is configured to receive the device(s) 120 in a position that allows the beads of the conductive thermal assembly 115 to substantially conform to at least a portion of the device(s) 120 geometry and to conduct heat to the device(s) 120. In an arrangement where the beads are coated with a solid form sterilant, heating of the beads in conjunction with reducing pressure in the chamber allows the sterilant that is encased in the polymer or other matrix to be released, thus sterilizing the device(s) 120. In an arrangement where the separate sterilant package 200 is provided, heating of the package 200 by the second heater 172 in conjunction with reducing pressure in the chamber 110 allows the sterilant that is encased in the package to be released.

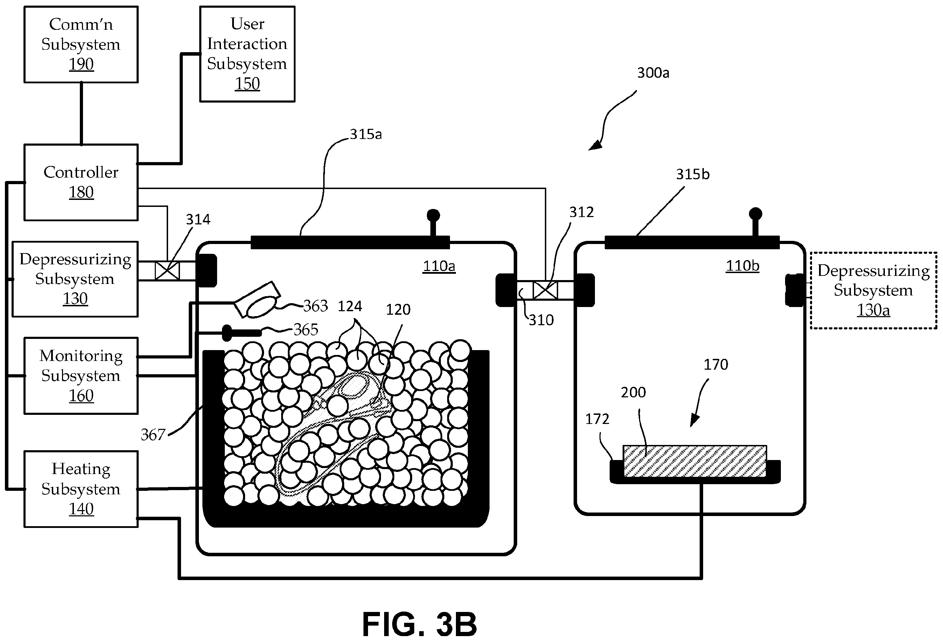

FIG. 3B illustrates another embodiment of a sterilizing system 300a. The sterilizing system 300a and its components are described using the same reference numbers as the embodiment of the sterilizing system 300 of FIG. 3A, where appropriate. This embodiment of the sterilizing system 300a is substantially similar to the sterilizing system 300 of FIG. 3A with the exception that this system 300a utilizes first and second separate vacuum or sterilizing chambers 110a and 110b. In this regard, the first chamber 110a (e.g., primary chamber) may include the conductive thermal assembly 115 configured to receive one or more medical devices 120. The second chamber 110b (e.g., antechamber) houses the sterilizing subassembly 170. As shown, the illustrated sterilizing subassembly 170 again includes a conductive heater 172 that supports a package 200 of solid-form sterilant (e.g., liquid sterilant disposed within a matrix). The heater 172 may heat the package 200 to a desired temperature within the second chamber 110b. The second chamber 110b may include a second door 315b, which allows a user to place the solid form sterilant therein.

In this embodiment, the first and second chambers 110a and 110b are in selective fluid communication. That is, these chambers 110a, 110b are connected via a fluid paths or conduit 310. In the present embodiment, the conduit 310 further includes valve 312 that may be actuated/controlled by the controller 180. Each of the illustrated embodiments includes a second valve 314 between the first chamber 110a and the pressurizing subsystem 130 (e.g., vacuum pump). Again, this valve 314 may be controlled by the controller 118. In operation, both valves 312, 314 may be opened to permit the depressurizing subsystem 130 to evacuate each chamber 110a, 110b to a desired vacuum. In various operations, upon achieving a desired pressure level (e.g., vacuum) the first valve 312 may be close to permit off-gassing of the sterilant from the sterilant package 200 into the second chamber 110b. The second valve 314 may remain open to permit continued evaporation and removal of water or other liquids from the medical device 120. When desired, the first valve 312 may be opened to permit adiabatic expansion of the gasified sterilant into the first chamber 110a. Additionally or optionally, the second valve 314 may be closed after water or other liquids are evaporated from the medical device and prior to opening the first valve to allow sterilant to enter into the main chamber 1 10a. In any case, such an arrangement permits adiabatic expansion of the gasified sterilant into the first chamber and into the medical device such that gasified sterilant is able to expand into all evacuated interior areas of the medical device. Such an arrangement may allow for reduced use of sterilant compared to a system that continually draws vacuum. Though illustrated as utilizing a single depressurizing system 130 for both chambers 110, 110b, it will be appreciated that the second chamber may have a separate depressurizing system 130a to permit, for example, separate depressurization (e.g., to a different vacuum level). Though discussed in relation to fluidly isolating the chambers, it will be appreciated that in some implementations, the chambers may remain in fluid communication throughout the process. In this implementation, the second chamber may primarily be used to control the separate heating of the solid-form sterilant.

The illustrated embodiments of the sterilizing systems 300 and 300a (hereafter 300 unless specifically referenced) include a monitoring subsystem 160 that can provide feedback control, environmental monitoring within the sterilizing chamber 110, monitoring of the device(s) 120, etc. The illustrated monitoring subsystem 160 includes one or more probes 365 (e.g., for monitoring internal pressure, humidity, and temperature within the sterilizing chamber 110) and one or more cameras 363 (e.g., for visualizing the internal environment of the sterilizing chamber 110 and/or visualizing the device(s) 120 before, during, and/or after the sterilizing routing). The illustrated monitoring subsystem 160 may also include an interface cable (not shown) for interfacing the sterilizing system(s) 300 with the device(s) 120 in the sterilizing chamber 110 (e.g., for sending and/or receiving communications between the controller 180 and the device(s) 120). The illustrated embodiment of the sterilizing system 300 can also include a disinfecting subsystem (not shown) for disinfecting the device(s) 120, the conductive thermal assembly 115, and/or the sterilizing chamber 110. As described above, the disinfecting subsystem can includes an ultraviolet lamp for irradiating the internal environment of the sterilizing chamber 110 to help kill contaminants. The various subsystems of the sterilizing system are in communication with a controller 180 through a bus or any other suitable wired or wireless link. For example, the controller 180 can be implemented as a central processing unit of the sterilizing system 300 or as a set of distributed processors, memories, etc.

A user can interact with functions of the sterilizing system 300 through a graphical user interface (GUI) and/or through any other user controls (e.g., buttons, switches, keypads, etc.). For example, the GUI is displayed on a display of the user interaction subsystem 150 or communication subsystem 190, which may include one or more touchscreens. In some implementations, the user interaction subsystem 150 provides minimal functionality (e.g., a button to begin the sterilizing process). In other implementations, the user interaction subsystem 150 provides complex functionality. For example, the user interaction subsystem 150 can display information, including multiple selections (e.g., soft buttons that provide the user with various options), routine progress (e.g., an estimated time remaining for completion), payment information (e.g., in a business setting), video feeds (e.g., from inside the sterilizing chamber 110, of the display of the device(s) 120, etc.), measured environmental levels (e.g., current readings of temperature, pressure, and/or humidity from within the sterilizing chamber 110), and/or any other useful information.

For the sake of illustration, a user with a contaminated medical device may utilize the sterilizing system 300. The user is may be presented with a GUI via the user interaction subsystem 150 that provides a number of selections, for example, "sterilize your device," or "disinfect your device." Each option has an associated time. The user selects one of the options. The door 315 of the sterilizing chamber 110 opens. The display instructs the user to place the device in or on the conductive thermal assembly 115. Additionally, a user may be instructed to connect the device to an interface cable (not shown). The display may further prompt the user to sign is for quality control, etc. When the sterilizing system 300 detects that the device is properly placed on or on the conductive thermal assembly 115 and/or connected to the interface cable, a soft button may appear on the GUI prompting the user to "press to start." The user touches the button. In response, the door 315 closes and locks (e.g., and seals) and the routine begins. The depressurizing subsystem 130 produces a sufficient vacuum within the sterilizing chamber 110 to gasify the water or liquids in and on the device, while the heating subsystem 140 conducts heat gently and evenly to the device via the beads or other heating system to support the sterilizing routine. Meanwhile, the display may show an elapsed time, an estimated remaining time, and a measured value of the temperature and humidity around the device in the chamber. The display can also show other useful information. When the routine completes, an indication is provided to the user (e.g., audible, visual, etc.). The user may further be prompted to insert credentials (e.g., any recognized form of identification, a code, etc.) prior to initiation the routine and/or upon completion of the routine. Once the sterilizing routine completes, a pressure valve releases, and the user is permitted to open the door 315 to retrieve the device.

Many other functions can be provided via the various subsystems in embodiments. For example, the user interaction subsystem 150 can be used to access maintenance, setup, diagnostics, debugging, and/or other functions. Further, the user interaction subsystem 150 can be used to receive various types of data from a user, like demographic information, product codes, etc. For example, implementations collect various types of Quality Management Systems (QMS) data and the like. Similarly, embodiments can collect operational information, such as frequency of use, frequency of success, cycle times, time since last maintenance, life time of the bead/zeolite sterilant emission system, error codes for diagnostics, etc. The data can be communicated (via the communications subsystem 190) to a host system (e.g., in the cloud, at a third-party location, etc.). Embodiments can also permit remote access (via the communications subsystem 190) for handling maintenance, diagnostics, updates, etc. In some implementations, QMS, billing tracking and/or other functions can be integrated with other systems. For example, if the sterilizing system is installed in a hospital, embodiments can integrate with hospital systems, such as the hospital's billing, QA, customer management, and/or other systems. For example, such a system may provide chain-of-custody information associated with a medical or other device.

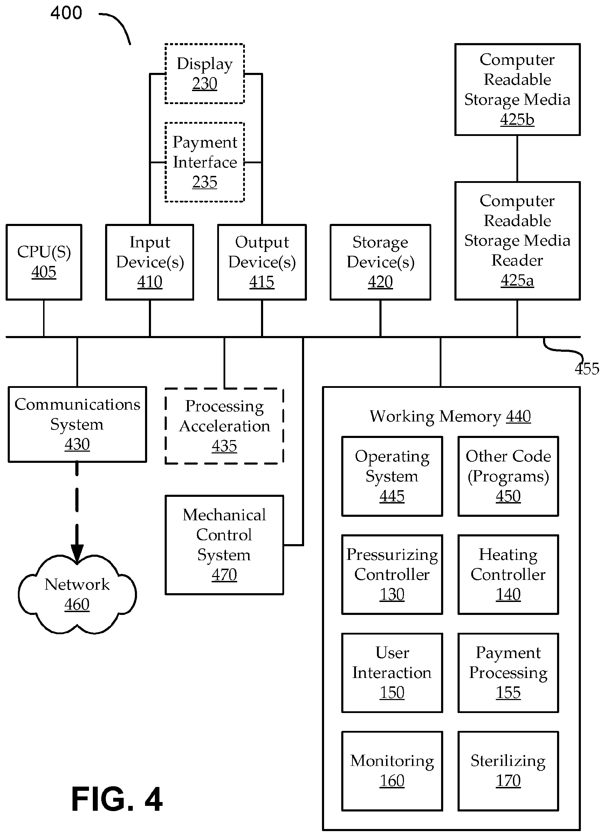

FIG. 4 shows an illustrative computational system 400 for implementing functionality of a sterilizing system, according to various embodiments. The computational system 400 can include or perform functionality of components of sterilizing system 105 embodiments, such as those described above in FIGS. 1 and 3. For the sake of simplicity, the computational system 400 is shown including hardware elements that can be electrically coupled via a bus 455. However, embodiments of the computational system 400 can be implemented as or embodied in single or distributed computer systems, in one or more locations, or in any other useful way.

The hardware elements can include one or more central processing units (CPUs) 405 (e.g., controller 180), one or more input devices 410 (e.g., a mouse, a keyboard, a display 230), and one or more output devices 415 (e.g., a display 230, a payment interface 335, if present, a coupon or receipt printer, etc.). The computational system 400 can also include one or more storage devices 420. By way of example, storage device(s) 420 can be disk drives, optical storage devices, solid-state storage device such as a random access memory (RAM) and/or a read-only memory (ROM), which can be programmable, flash-updateable and/or the like.

The computational system 400 can additionally include a computer-readable storage media reader 425a, a communications system 430 (e.g., communications subsystem 190, including a modem, a network card (wireless or wired), an infra-red communication device, etc.), and working memory 440, which can include RAM and ROM devices as described above. In some embodiments, the computational system 400 can also include a processing acceleration unit 435, which can include a DSP, a special-purpose processor, and/or the like.

The computer-readable storage media reader 425a can further be connected to a computer-readable storage medium 425b, together (and, optionally, in combination with storage device(s) 420) comprehensively representing remote, local, fixed, and/or removable storage devices plus storage media for temporarily and/or more permanently containing computer-readable information. The communications system 430 can permit data to be exchanged with a network 460 and/or any other computer described above with respect to the computational system 400. For example, as described with reference to FIGS. 1 and 3, product information, CRM data, remote diagnostics, and/or other information can be communicated to and from the computational system 400 via the communications system 430 to the network 460.

The computational system 400 can also include software elements, shown as being currently located within a working memory 440, including an operating system 445 and/or other code 450, such as an application program (which can be a client application, web browser, mid-tier application, relational database management system (RDBMS), etc.). In some embodiments, one or more functions of the subscriber optimizer 120 are implemented as application code 450 in working memory 440. For example, as illustrated, depressurizing functionality 130, heating functionality 140, user interaction functionality 150, product processing functionality 155, monitoring functionality 160, disinfecting/sterilization functionality 170, etc. can be implemented as code of the working memory 440 (e.g., as part of the other code 450). Some embodiments further include a mechanical control system 470 to control various mechanical (e.g., electromechanical) features of the computational system 400. For example, the mechanical control system 470 can fully or partially control operation of the conveyor assembly 125, the door 315 to the sterilizing chamber 110, motion of the sterilizing chamber 110, etc.

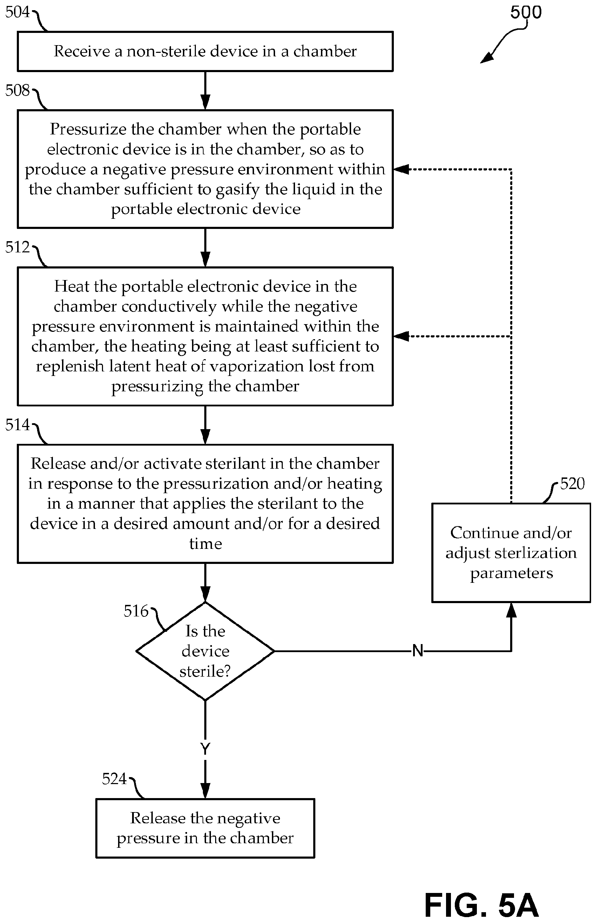

FIG. 5A shows a flow diagram of one illustrative method 500 for sterilizing an electronic medical device, non-electronic medical devices, instruments and other medical items 120, according to various embodiments. The method 500 operates in context of sterilizing systems, such as those described above with reference to FIGS. 1-4. Embodiments begin at stage 504, by receiving a device 120 (e.g., a portable electronic medical device, non-electronic medical device, instrument, etc.) in a chamber. As described above, it is assumed that the device is non-sterile. For example, the device is scheduled for sterilization, assumed to need sterilization (e.g., after a procedure, in accordance with a policy or standard, etc.), the device is known to have an excessive amount of contamination in (and possibly on) the device, etc. The device can be placed in the chamber through a door or other scalable opening in the chamber. Typically, the device is placed into contact with a thermal conduction assembly or the device and/or the thermal conduction assembly are moved into contact with each other as the method 500 begins (e.g., when the chamber door is closed, etc.).

At stage 508, the chamber is depressurized when the device(s) 120 are in the chamber, so as to produce a negative pressure environment (e.g., a substantial vacuum) within the chamber sufficient to gasify the liquid in the device(s) 120. For example, the chamber is fluidly coupled with a vacuum pump. When the vacuum is established and the liquid gasifies, latent heat of vaporization is lost.

At stage 512, the device(s) 120 are conductively heated in the chamber (e.g., via a thermal conduction assembly, such as beads) while the negative pressure environment is maintained within the chamber. The heating is at least sufficient to replenish the latent heat of vaporization lost from pressurizing the chamber. As described above, any suitable type of thermal conduction assembly can be used. Further the thermal conduction assembly may at least partially conform to an external shape of the device(s) 120.

At stage 514, depressurization at stage 508 and/or heating at stage 512 causes sterilant in the chamber to be released and/or activated in a manner that applies the sterilant to the device(s) 120 in a desired amount and/or for a desired time. For example, the depressurization at stage 508 causes vaporization or off-gassing of encapsulated liquid sterilant that is coating the beads of a thermal conduction assembly of the heated in stage 512. Alternatively, the depressurization at stage 508 causes vaporization or off-gassing of encapsulated liquid sterilant in a package within the chamber.

In some implementations, at stage 516, a determination is made as to whether the excess contamination has been removed from the device(s) 120 (e.g., whether the device is sufficiently sterile). If not, one or more steps can be taken, such as maintaining and/or adjusting the sterilization parameters (e.g., negative pressure and/or the heating within the chamber). If so, at stage 524, the chamber can be vented (e.g., via a release valve) to return the chamber to atmospheric pressure.

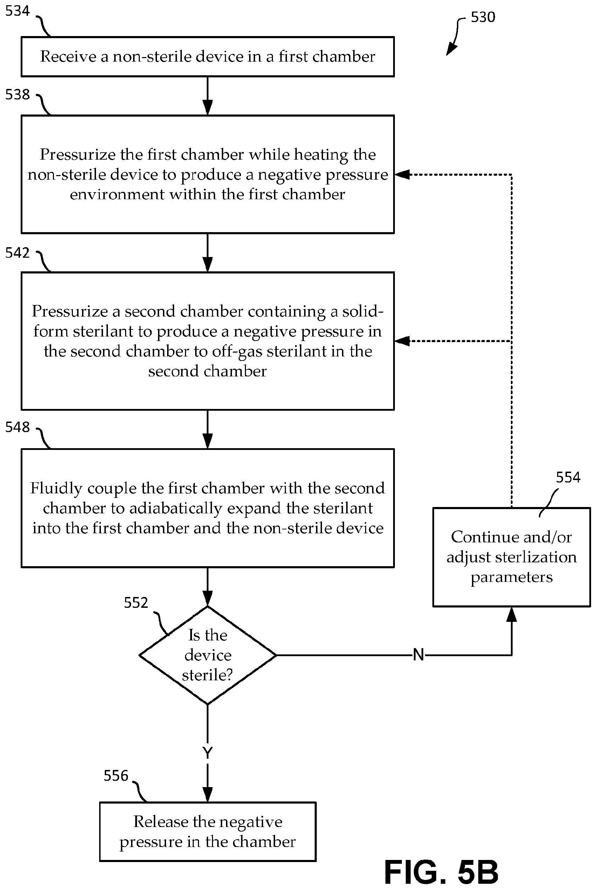

FIG. 5B shows a flow diagram of another illustrative method 530 for sterilizing an electrical medical device, non-electronic medical devices, instruments and other medical items 120, according to various embodiments. The method 530 begins at stage 534, by receiving a device (e.g., electronic medical device, non-electronic medical device, instrument, etc.) in a first chamber of a two chamber system. Typically, the device is placed into contact with a thermal conduction assembly of the device and/or the thermal conduction assembly are moved into contact with each other as the method 530 begins (e.g., when the chamber door is closed, etc.). At stage 538, the first chamber is depressurized to produce a negative pressure environment (e.g., a substantial vacuum) within the first chamber in conjunction with heating the device. At stage 542, a second chamber, containing a solid-form sterilant (e.g., sterilant package), is depressurized to produce a negative pressure environment to off-gas (gasify or vaporize) at least a portion of liquid sterilant within the solid-form sterilant package. Stage 542 may further include heating the sterilant package. Further stages 538 and 542 may be performed simultaneously using a common depressurizing system. Once the chambers reach a desired negative pressure environment, the first and second chambers may be fluidly coupled to permit the gasified sterilant in the second chamber to adiabatically expand into the first chamber at stage 548. The fluidly coupling of the chambers may occur after a predetermined time to permit, for example, complete drying of a device in the first chamber. Along these lines the chambers may be fluidly isolated after or during depressurizing. In some implementations, at stage 552, a determination is made as to whether contamination has been removed from the device(s) 120 (e.g., whether the device is sufficiently sterile). If not, one or more stages can be repeated 554 (e.g., reducing pressure in one or both chambers, fluidly coupling the chambers etc.). That is, the process may be repeated to repeatedly purge the device and allow for repeated expansion of gasified sterilant into the device. If sufficient sterilization is achieved, at stage 556, the negative pressure in the chamber can be released (e.g., via a release valve).

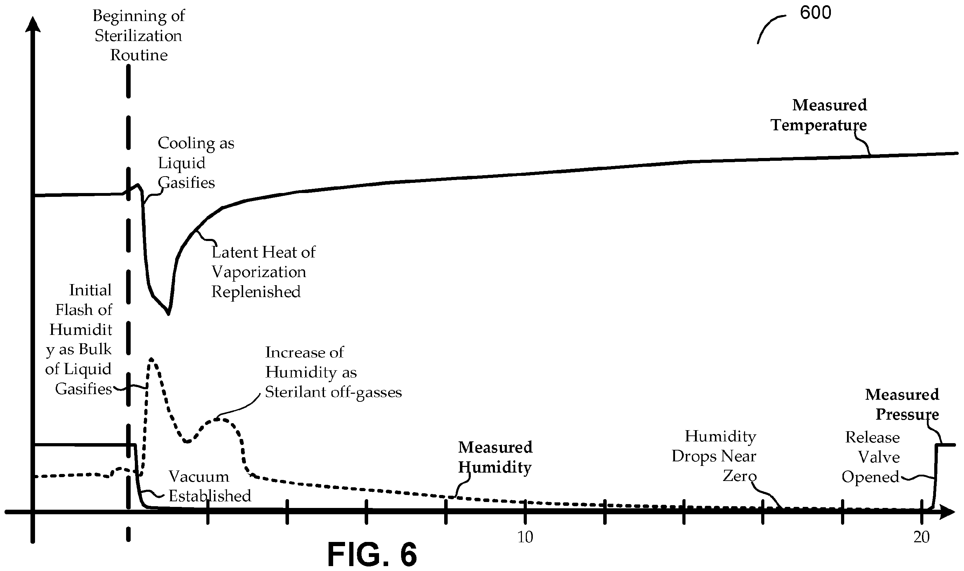

For the sake of illustration, FIG. 6 shows a graphical representation 600 of an illustrative sterilizing cycle. Three traces are shown, representing temperature, pressure, and humidity levels measured within the sterilizing chamber (e.g., adjacent to the device(s) 120) over time. For example, the cycle time is shown as approximately twenty minutes. A vertical dashed line indicates the beginning of the sterilizing routine. As illustrated by the "measured pressure" trace, the system pressure begins at a "normal" atmospheric level, and quickly drops when the routine begins and a vacuum is established (producing a negative pressure environment) in the sterilizing chamber. The desired vacuum level is substantially maintained until a release valve is opened at the end of the routine and pressure in the chamber returns to the normal atmospheric level. As illustrated by the "measured humidity" trace, the humidity in the sterilizing chamber increases dramatically as the vacuum is first established and the bulk of the contamination in the device(s) 120 gasifies (e.g., boils off). After that initial spike, the measured humidity in the chamber begins to drop, raises slightly (not to scale) as the encased liquid sterilant from the beads/zeolites begins to be released. The process shown illustrates continuous operation of the depressurization system. However, it will be appreciated that the depressurization system may be shut off for a period of time once the monitored humidity (or possibly pressure) begins to rise due to the evaporation of the sterilant. This may permit more expansion of the sterilant and further sterilization. After a specific amount of time in the gasified liquid sterilant field, the device(s) 120 are deemed sterilized. Once sterilized, the chamber may be further evacuated and/or returned to atmospheric pressure.

As illustrated by the "measured temperature" trace, the temperature in the sterilizing chamber decreases as the vacuum is first established, and the latent heat of vaporization is lost from gasification of the liquid. After the initial decrease in temperature, conductive heat applied to the device(s) 120 gently replenishes the lost heat throughout the remainder of the routine, at least as desired

As described above, the sterilant package or potentially coated beads contain a liquid sterilant that are encapsulated in a stabilizing matrix. The stabilizing matrix that containing the sterilant is called the sterilant matrix. The sterilant matrix and encapsulated sterilant is ultimately placed in the sterilizing system (e.g., sterilization chamber), to undergo the sterilizing routine in which the chamber is pressurized to a vacuum level sufficient to gasify the sterilant within the sterilant matrix.

The sterilant is initially stabilized in a matrix material where the sterilant molecules are reversibly trapped in the interior of the matrix to prevent reaction and to suppress degradation. The stabilization matrix may consist of synthetic polymers, biopolymers, ceramics, glass, or composite materials, or any combination of these materials. Some examples of synthetic polymers that may be used include, but are not limited to, silicones, polyacrylates, polyethylenes and related polymers, polyamides, polyurethanes, polyethers, polyphosphazenes, polyanhydrides, polyacetals, poly (ortho esters), polyphosphoesters, polycaprolactones, polylactides, and polycarbonates. Some examples of biopolymers that may be used include, but are not limited to, carbohydrates, starches, celluloses, chitosans, chitins, dextrans, gelatins, lignins and polyamine acids. Some examples of ceramics include, but are not limited to, aluminum oxide, zirconium oxide, silicon dioxide, magnesium oxide, titanium oxide, aluminum nitride, silicon nitride, boron nitride and silicon carbide. Composite materials may consist, but are not limited to, two or more of the matrix materials listed above.

The sterilant is typically prepared in liquid form (e.g., in situ) and then mixed or otherwise loaded into the stabilization matrix. The sterilant matrix is used to form a gel or a solid that contains the sterilant molecules. The sterilant gel or solid may be shaped into the form of beads, a block and/or may be stored in a container or wrapping that is sufficiently porous and such that allows the sterilant vapor molecules to outgas from the material matrix and/or container when exposed to reduced pressure and/or increased temperature. The purity and concentration of sterilant loaded into the matrix material can be adjusted according to the intended use. It may be seen that a sterilant gel or solid that contains a purer and more concentrated form of sterilant may be used for multiple sterilization runs, for killing more resistant microbes, and/or for sterilizing a larger bioload. The sterilizing system can be configured for a user to easily and safely place the sterilant gel or solid into the chamber before depressurizing the system and prior to initiating a sterilization cycle. The encapsulation of sterilant in a matrix material may also provide a simplified method for extending the shelf-life of the sterilant in such a way that the sterilant does not need to be prepared just prior to use. The encapsulated sterilant may sit at or near temperatures without spontaneously degrading for an extended period giving the user the opportunity to pull the sterilant gel or solid off of the self when needed to insert it into the sterilizing system for one or more uses.

As noted, the encapsulated sterilant (e.g., solid-form sterilant) may be provided in a container or wrapper. In one implementation, the sterilant may be provided in a gas permeable packet. In such an implementation, the solid-form sterilant may be provided in, for example vapor gas porous polytetrafluoroethlyne (PTFE), polyethersulfone (PES) or high-density polyethylene (HDPE) membranes, to name a few. The exact gas-permeable membrane selected may be based on the sterilant and/or matrix material of a given solid-form sterilant. In some implementations, the solid-form sterilant may be disposed within a sealed gas-permeable membrane and then sealed within a non-permeable wrapper. Prior to use, the non-permeable wrapper may be removed and the solid-form sterilant within the gas permeable membrane may be inserted into the chamber or antechamber of the sterilizing system. Alternatively, the solid-form matrix may be removed from a non-permeable container or wrapper and placed directly within a sterilizing system.

In various implementations, it may be desirable to heat the solid-form sterilant to a temperature that is elevated compared to a temperature that a medical device is heated (e.g. to replace the latent heat of vaporization lost during the pressurization. For instance, many electrical devices may degrade at temperatures above about 30 degrees Celsius. However, depending on the matrix material and sterilant, a solid-form sterilant may require or better vaporize (e.g., off gas) at higher temperatures. Accordingly, by providing separate heaters and/or chambers, the temperatures of the medical device and sterilant may be individually controlled. This may provide improved sterilizing performance.

The use of the solid-form sterilant was found to provide significantly improved performance in comparison with the use of liquid sterilants. It is believed that liquid sterilants evaporate too quickly in negative pressure environments. Along these lines, it was determined that use of liquid sterilants, to achieve substantially equal levels of sterilization, required a significant increase in the amount of sterilant utilized, which, in some instances raised concerns about corrosion.

The following examples are intended to be illustrative only.

### EXAMPLE A - STERILIZATION OF A SURGICAL SCALPEL USING HYDROGEN PEROXIDE/PEG SOLID STERILANT

In this example, a mixture of 50/50 W/V polyethylene glycol 3350 (Sigma Aldrich, St. Louis, Mo) / 30% hydrogen peroxide (Sigma Aldrich, St. Louis, MO) is created using 0.5g of each. This mixture is stirred at 300 RPM on a heated stir plate at 30⁰C for 30 minutes. The mixture is then transferred to a mold to solidify.

The solid sterilant, scalpel, and, for each run, ampoules charged with a *Geobacillus stearothermophilus* spore density of 2.1 × 10⁶ spores/unit were placed in sterilization chamber: 1) on top of aluminum beads, in the front and the back of the chamber, and 2) 5 inches below surface of the aluminum beads, in the front and the back of the chamber. The chamber is then evacuated to 1×10⁻³ Torr while heat of 40°C is evenly applied to the chamber. Once the base pressure is reached, the system is allowed to remain at a steady state of about 1 Torr for 15 minutes. The presence of reactive oxygen species vapor was measured qualitatively using a colorimetric indicator. Colorimetric indicators were also placed in the sterilization chamber: 1) on top of the aluminum beads, in the front and back of the chamber, and 2) 5 inches below surface of the aluminum beads, in the front and back of the chamber. After all spore ampoules, medical device and indicators were in place the chamber, it was closed and the sterilization run was initiated. At the completion of the 15 minute sterilization cycle the chemical indicators and the spore ampoules were removed from the chamber. The spore ampoules were incubated at 58°C for 24 hours and then tested for an SAL of 10⁻⁶ spores/unit. The ampules were found to be acceptably sterile at the 10⁻⁶ level.

### EXAMPLE B - STERILIZATION OF A SURGICAL SCALPEL USING HYDROGEN PEROXIDE LIQUID STERILANT

In this example 5g of 30% liquid hydrogen peroxide (Sigma Aldrich, St. Louis, MO) is introduced into the chamber.

The sterilant, scalpel, and, for each run, ampoules charged with a *Geobacillus stearothermophilus* spore density of 2.1 × 10⁶ spores/unit were placed in sterilization chamber: 1) on top of aluminum beads, in the front and the back of the chamber, and 2) 5 inches below surface of the aluminum beads, in the front and the back of the chamber. The chamber is then evacuated to 1×10⁻³ Torr while heat of 40°C is evenly applied to the chamber. Once the base pressure is reached, the system is allowed to remain at a steady state of about 1 Torr for 15 minutes. The presence of reactive oxygen species vapor was measured qualitatively using a colorimetric indicator. Colorimetric indicators were also placed in the sterilization chamber: 1) on top of the aluminum beads, in the front and back of the chamber, and 2) 5 inches below surface of the aluminum beads, in the front and back of the chamber. After all spore ampoules, medical device and indicators were in place the chamber, it was closed and the sterilization run was initiated. At the completion of the 15 minute sterilization cycle the chemical indicators and the spore ampoules were removed from the chamber. The spore ampoules were incubated at 58°C for 24 hours and then tested for an SAL of 10⁻⁶ spores/unit. The ampules were found to **NOT** be acceptably sterile at the 10⁻⁶ level.

This result is surprising as this example used ten times the amount of sterilant as the above example, yet the SAL of 10⁻⁶ was not achieved.

### EXAMPLE C - STERILIZATION OF A SURGICAL SCALPEL USING HYDROGEN PEROXIDE/PEG SOLID STERILANT AND MULTIPLE HEATING ZONES

In this example, a mixture of 50/50 W/V polyethylene glycol 3350 (Sigma Aldrich, St. Louis, Mo) / 30% hydrogen peroxide (Sigma Aldrich, St. Louis, MO) is created using 0.5g of each. This mixture is stirred at 300 RPM on a heated stir plate at 30⁰C for 30 minutes. The mixture is then transferred to a mold to solidify.

The chamber was modified to include an antechamber that is connected to the main sterilization chamber vis a ¼"stainless steel tube with a manual leak valve that allows controlled flow from the antechamber to the main chamber. The antechamber and main chamber have different, independently controlled heating.

The solid sterilant is inserted into the antechamber, and the scalpel, and ampoules charged with a *Geobacillus stearothermophilus* spore density of 2.1 × 10⁶ spores/unit were placed in sterilization chamber: 1) on top of the aluminum beads, in the front and the back of the chamber, and 2) 5 inches below surface of the aluminum beads, in the front and the back of the chamber. The sterilization chamber is then evacuated to 1×10⁻³ Torr while heat of 40⁰C is evenly applied to the chamber and a temperature of 60⁰C is applied to the antechamber. Once the base pressure is reached, the antechamber is slowly opened and is allowed to remain at a steady state of about 1 Torr for 15 minutes. The presence of reactive oxygen species vapor was measured qualitatively using a colorimetric indicator. Colorimetric indicators were also placed in the sterilization chamber: 1) on top of the aluminum beads, in the front and back of the chamber, and 2) 5 inches below surface of the aluminum beads, in the front and back of the chamber. After all spore ampoules, medical device and indicators were in place the chamber, it was closed and the sterilization run was initiated. At the completion of the 15 minute sterilization cycle the chemical indicators and the spore ampoules were removed from the chamber. The spore ampoules were incubated at 58°C for 24 hours and then tested for an SAL of 10⁻⁶ spores/unit. The ampules were found to be acceptably sterile at the 10⁻⁶ level.

### EXAMPLE C - STERILIZATION OF A SURGICAL SCALPEL USING HYDROGEN PEROXIDE/PEG SOLID STERILANT AND MULTIPLE HEATING ZONES AND A PULSED STERILIZATION

In this example, a mixture of 50/50 W/V polyethylene glycol 3350 (Sigma Aldrich, St. Louis, Mo) / 30% hydrogen peroxide (Sigma Aldrich, St. Louis, MO) is created using 0.5g of each. This mixture is stirred at 300 RPM on a heated stir plate at 30⁰C for 30 minutes. The mixture is then transferred to a mold to solidify.

The chamber was modified to include an antechamber (e.g., second chamber) that is connected to the main sterilization chamber vis a ¼"stainless steel tube with a manual leak valve that allows controlled flow from the antechamber to the main chamber. The antechamber and main chamber have different, independently controlled heating.

The solid sterilant is inserted into the antechamber, and the scalpel, and ampoules charged with a *Geobacillus stearothermophilus* spore density of 2.1 × 10⁶ spores/unit were placed in sterilization chamber: 1) on top of the aluminum beads, in the front and the back of the chamber, and 2) 5 inches below surface of the aluminum beads, in the front and the back of the chamber. The sterilization chamber is then evacuated to 1×10⁻³ Torr while heat of 40⁰C is evenly applied to the chamber and a temperature of 60⁰C is applied to the antechamber. Once the base pressure is reached, the pumping valve to the sterilization chamber is closed and the antechamber is slowly opened and is allowed to remain at a steady state of about 4 Torr for 5 minutes. The antechamber valve is then closed, and the pumping valve for the sterilant chamber is oven and the chamber is evacuated. Once the sterilization chamber is evacuated to 1×10⁻³ Torr, the pumping valve is again closed and the valve to the antechamber is again open and allowed to remain at a steady state of 4 Torr for 5 minutes. This process is then repeated a total of 3 times for a total of 15 minutes of total sterilization time.

The presence of reactive oxygen species vapor was measured qualitatively using a colorimetric indicator. Colorimetric indicators were also placed in the sterilization chamber: 1) on top of the aluminum beads, in the front and back of the chamber, and 2) 5 inches below surface of the aluminum beads, in the front and back of the chamber. After all spore ampoules, medical device and indicators were in place the chamber, it was closed and the sterilization run was initiated. At the completion of the sterilization cycles the chemical indicators and the spore ampoules were removed from the chamber. The spore ampoules were incubated at 58°C for 24 hours and then tested for an SAL of 10⁻⁶ spores/unit. The ampules were found to be acceptably sterile at the 10⁻⁶ level.

### EXAMPLE D - STERILIZATION OF A SURGICAL SCALPEL USING HYDROGEN PEROXIDE/PEG COATED THERMAL CONDUCIVE BEADS

In the second example, aluminum beads of diameter 7 mm can be coated with a mixture of 50/50 W/V polyethylene glycol 3350 (Sigma Aldrich, St. Louis, Mo) / 30% hydrogen peroxide (Sigma Aldrich, St. Louis, MO) using the bead coating method described above in the spraying application gun method and the low-pressure atomization method. In this system liquid is pumped through an orifice of 0.045 inch at relatively low pressure of 35 psig. Low pressure of 55 psig at the atomizer tip can be used. The beads are preheated for 20 minutes at 30⁰C and sprayed with the mixture set at 30°C and drying heat at 30⁰C for 20 minutes creating a coating of about 200 microns evenly distributed over the surface of all the beads.

The beads, scalpel, and SAL test strip are then placed in the sterilization chamber as described above. The chamber is then evacuated to 1×10⁻³ Torr while heat of 40⁰C is evenly applied to the chamber. Once the base pressure is reached, the system is allowed to remain at a steady state of about 1 Torr for 15 minutes. At the end of the 15 minutes, the chamber is vented with argon gas until it reaches atmospheric pressure. The scalpel and SAL test strip is then removed using well established sterile techniques. The SAL strip is then tested and will be found to be acceptably sterile at the 10⁻⁶ level.

### EXAMPLE E - STERILIZATION OF AN ENDOSCOPE USING THERMALLY CONDUCTIVE ZEOLITES SATURATED WITH CHLORINE DIOXIDE

The fourth example involves the sterilization of an electronic medical device, namely an endoscope. Natural zeolites (Clinoptilolite) of diameter 5-10 mm (Newstone International Corporation, Tokyo) can be infused with chlorine dioxide (Sigma Aldrich, St. Louis, MO) using the zeolite coating method described above in the fluid bed coating method at -5⁰C. In this system liquid is pumped through an orifice of 0.045 inch at relatively low pressure of 35 psig. Low pressure of 55 psig at the atomizer tip can be used. The zeolites are pre-chilled for 20 minutes at -5⁰C and sprayed with the mixture set at -10⁰C and drying heat at -5⁰C for 20 minutes creating an infused zeolite.

The zeolites, endoscope, and SAL test strip are then placed in the sterilization chamber as described above. The chamber is then evacuated to 1×10⁻³ Torr while heat of 15⁰C is applied evenly to the chamber. Once the base pressure is reached, the system is allowed to remain at a steady state of about 1 Torr for 15 minutes. At the end of the 15 minutes, the chamber is vented with argon gas until it reaches atmospheric pressure. The endoscope and SAL test strip is then removed using well established sterile techniques. The SAL strip is then tested and will be found to be acceptably sterile at the 10⁻⁶ level.

### EXAMPLE F - STERILIZATION OF A MEDICAL SMART TABLET THROUGH THE USE OF PHOTO-ACTIVATED RIBOFLAVIN /PEG COATED BEADS USING A ULTRAVIOLET LAMP

In the fifth example, glass beads of diameter 20 mm can be coated with a mixture of 50/50 W/V polyethylene glycol 3350 (Sigma Aldrich, St. Louis, Mo) / photo-activated riboflavin (Sigma Aldrich, St. Louis, MO) using the bead coating method described above in the spraying application gun method and the low-pressure atomization method. In this system liquid is pumped through an orifice of 0.045 inch at relatively low pressure of 35 psig. Low pressure of 55 psig at the atomizer tip can be used. The beads are preheated for 20 minutes at 30⁰C and sprayed with the mixture set at 30°C and drying heat at 30⁰C for 20 minutes creating a coating of about 200 microns evenly distributed over the surface of all the beads.

The beads, medical smart tablet, and SAL test strip are then placed in the sterilization chamber as described above. The chamber is then evacuated to 1×10⁻³ Torr while heat of 40⁰C is evenly applied to the chamber. The UV light that is attached to the sterilization subsystem inside the chamber is illuminated once the base pressure is reached. The beads being made of glass, and the bead coating being PEG, the UV light is able to penetrate the entire system, thus activating the photo-activated riboflavin.

Once the base pressure is reached, the system is allowed to remain at a steady state of about 1 Torr for 15 minutes. At the end of the 15 minutes, the chamber is vented with argon gas until it reaches atmospheric pressure. The scalpel and SAL test strip is then removed using well established sterile techniques. The SAL strip is then tested and will be found to be acceptably sterile at the 10⁻⁶ level.

### EXAMPLE G - STERILIZATION OF A SCALPEL THROUGH THE USE OF CIDEX INFUSED ZEOLITES AND A VIBRATORY SUBSYSTEM TO ENSURE EVEN DISTRIBUTION OF STERILANT

The sixth example involves the sterilization of a scalpel. Natural zeolites (Clinoptilolite) of diameter 5-10 mm (Newstone International Corporation, Tokyo) can be infused with the chemical sterilant CIDEX containing 2.4% glutaraldehyde (ASP, a Johnson and Johnson company, New Jersey, USA) using the zeolite coating method described above in the fluid bed coating method at 20⁰C. In this system liquid is pumped through an orifice of 0.045 inch at relatively low pressure of 35 psig. Low pressure of 55 psig at the atomizer tip can be used. The zeolites are pre-chilled for 20 minutes at 20⁰C and sprayed with the mixture set at 20°C and drying heat at 35°C for 20 minutes creating an infused zeolite.

The zeolites, scalpel, and SAL test strip are then placed in the sterilization chamber as described above. The chamber is then evacuated to 1×10⁻³ Torr while heat of 30⁰C is applied evenly to the chamber. Once the base pressure is reached, the vibratory subsystem is started and the system is allowed to remain at a steady state of about 1 Torr for 15 minutes. At the end of the 15 minutes, the chamber is vented with argon gas until it reaches atmospheric pressure. The scalpel and SAL test strip is then removed using well established sterile techniques. The SAL strip is then tested and will be found to be acceptably sterile at the 10⁻⁶ level.

In another implementation, Performic acid is encapsulated in a matrix to produce a solid-form sterilant having a shelf life considerably in excess of liquid Performic acid. Performic acid liquid is an effective sterilizing agent that is also a highly successful sterilant in the form of a vapor. Other effective sterilizing agents can be vaporized from a mixture containing Performic acid in addition to, but not limited to, any number of other saturated and unsaturated peroxy acids having between 1 and 8 carbon atoms and including any halogenated forms of the peracids, so long as the molecule(s) being utilized are adequately volatilized to form a vapor of a concentration necessary to sterilize the device in question under reduced pressures in a vacuum sterilization systems as described above, with or without the assistance of heat at a temperature of equal to or less than what is required by the device being sterilized. Some examples of other peracids include, but are not limited to, peroxyacetic acid and its halogenated derivatives, peroxypropionic acid, and its halogenated derivatives and peroxybutyric acid and its halogenated derivatives. Halogenated peracids may contain one or more fluoro-, chloro-, bromo- and/or iodo- groups. Other sterilizing agents that may also be vaporized as part of a mixture include, but are not limited to, the peracid parent acid, hydrogen peroxide, and alcohols.

Some embodiments described herein use performic acid as the peracid due to its volatility, although there are others that will suffice. Some advantages of using performic acid as a sterilant over other chemicals for use herein are described below. Performic acid has a higher vapor pressure (78 mmHg @ 25°C) over its commonly used counterpart peracetic acid (14.5 mmHg @ 25°C), making it easier and faster to vaporize, allowing for an accelerated sterilization run. Other commonly used sterilants have several disadvantages. Hydrogen peroxide and ozone are stronger oxidizers than performic acid and, as such, may damage device materials being sterilized. Hydrogen peroxide also attacks commonly used sterilization packaging materials, such as cellulose. The breakdown products of performic acid are carbon dioxide and water whereas those of peracetic acid are acetic acid and water. The corrosive by-products of peracetic acid may limit its use on some devices. Performic acid is non-toxic, and there are no reports of tumorigenic properties. The bactericidal and sporicidal properties of peracetic acid, peformic acid, and perpropionic acids have been documented, and of the three peroxyacids, performic acid activity as an anti-fungicide surpasses that of its counterparts. Performic acid's mode of action, as an active oxidizing agent for killing microbial cells, is a highly effective and fast acting process of cleaving disulfide bonds of microbial cells ultimately causing death of the cell. Also, the sterilization action of performic acid is faster and has a lower concentration threshold than that of related peracid compounds such as peracetic acid. The Association of Official Analytical Chemists (AOAC) completed a Sporicidal Challenge and found that the D-value of performic acid may be lower than five minutes at a low concentration of 1,800 ppm at 44°C.

Solutions of aqueous performic acid, up to 90% pure, are most commonly prepared by mixing commercially available formic acid (70-98 wt %) with hydrogen peroxide (35-50 wt %). Performic acid, which is not commercially available, is unstable and decomposes immediately upon standing and must be used within a limited time after synthesis. As a result, solutions of formic acid and hydrogen peroxide must be mixed just prior to use (in situ). The highly reactive nature of performic acid when heated, the limited amount of time one must utilize the sterilization solution once synthesized, and the hazards of handling unstable liquid solutions are several drawbacks to using performic acid in previous methods that use sterilant vapors.

Embodiments described herein provide systems and methods for using performic acid, or any combination of performic acid and other liquid to generate sterilant vapors in a sterilization chamber at reduced pressure by avoiding the drawbacks described above. Embodiments make use of peracid vapors, especially performic acid vapors, to sterilize a number of different medical, dental and other devices by way of providing methods that allow the user to safely provide control over introducing the unstable and reactive liquid sterilizing agent into a vacuum sterilization system.

In one embodiment, these sterilizing agents are vaporized, preferably, from a liquid containing performic acid or a mixture of performic acid with other peracids and/or solutions that are encapsulated in a stabilizing matrix. The performic acid can be stabilized in a material matrix which may consist of synthetic polymers, biopolymers, ceramics, glass, or composite materials, or any combination of these materials. The performic acid is prepared in liquid form in situ and then immediately mixed or loaded into the stabilization matrix. The sterilant matrix is used to form a gel or a solid that contains the performic acid molecules as discussed above. This embodiment provides a simplified method for extending the shelf-life of the performic acid in such a way that the sterilant does not need to be prepared just prior to use. It may sit at a reasonable temperature without spontaneously degrading for an extended period giving the user the opportunity to pull the sterilant gel or solid off of the self when needed to insert it into the sterilizing system for one or more uses. When contained in a stabilization matrix, performic acid can be safely produced in bulk for use within sterilizing systems, such as those described herein.

Performic acid used in the examples listed below can be synthesized by mixing Hydrogen Peroxide, 30 wt% (Sigma Aldrich, St. Louis, MO) with Formic Acid, 90% (Sigma Aldrich, St. Louis, MO). The vapors of performic acid are generated according to techniques, such as those described herein.

### EXAMPLE H - METHOD FOR PREPARING STERILIZATION GEL FROM PERFORMIC ACID AND POLYETHYLENE GLYCOL

A mixture of 50/50 w/w polyethylene glycol 1000 (Sigma Aldrich, St. Louis, MO) and performic acid (Sigma Aldrich, St. Louis, MO) is created. This mixture is stirred at 300 RPM on a stir plate at room temperature for 30 minutes. The gel is then transferred to a container or wrapping sufficiently porous to emit vapors.

### EXAMPLE I - STERILIZATION OF A SURGICAL SCALPEL USING PERFORMIC ACID STERILIZATION GEL

The gel from example 1, scalpel and a self-contained biological indicator ampoule (MesaLabs, Bozeman, MT) containing a SAL strip with a spore Geobacillus stearothermophilus population of 106 are placed in the sterilization chamber. The chamber is then evacuated to 1X10-3 Torr while heat of 40°C is evenly applied to the chamber. Once the base pressure is reached, the system is allowed to remain in a steady state of about 1 Torr for 15 minutes. At the end of the 15 minutes, the chamber is vented with argon gas until it reaches atmospheric pressure. The scalpel and biological indicator ampoule are removed using well-established sterile techniques. The SAL strip is tested within the biological indicator by gently crushing ampoule and then incubating the biological indicator ampoule for 24 hours at 60°C. Following time, it will be found to be acceptably sterile at the 10⁻⁶ level.

### EXAMPLE J - METHOD FOR PREPARING STERILIZATION SOLID FROM PERFORMIC ACID AND POLYETHYLENE GLYCOL

A mixture of 85/15 w/w polyethylene glycol 3350 (Sigma Aldrich, St. Louis, MO) and polyethylene glycol 1000 (Sigma Aldrich, St. Louis, MO) is created by melting both polyethylene glycols in a glass beaker at 58°C while stirring at 300 RPM on a heating stir plate for 30 minutes. After time let mixture cool to room temperature. The 85/15 w/w polyethylene glycol solid is compounded with performic acid to form a solid that contains 25 wt% performic acid.

### EXAMPLE K - STERILIZATION OF SURGICAL SCALPEL USING PERFORMIC ACID SOLID

The solid from example 2, scalpel, and a self-contained biological indicator ampoule (MesaLabs, Bozeman, MT) containing a SAL strip with a spore Geobacillus stearothermophilus population of 106 are placed in the sterilization chamber. The chamber is then evacuated to 1X10-3 Torr while heat of 40°C is evenly applied to the chamber. Once the base pressure is reached, the system is allowed to remain in a steady state of about 1 Torr for 15 minutes. At the end of the 15 minutes, the chamber is vented with argon gas until it reaches atmospheric pressure. The scalpel and biological indicator ampoule are removed using well-established sterile techniques. The SAL strip is tested within the biological indicator by gently crushing ampoule and then incubating the biological indicator ampoule for 24 hours at 60°C. Following time, it will be found to be acceptably sterile at the 10⁻⁶ level.

As set forth above, in various implementations, the solid form sterilant may be applied to the beads of the thermal conductive assembly. Various techniques can be used to implement sterilant-coated beads or zeolites. For example, a spray and tumble apparatus can be used to coat the beads and zeolites. Bead/Zeolite coating can generally refer to application of coating composition to a moving bed of the beads / zeolites with concurrent use of heated air to facilitate evaporation of the solvent.

In some implementations, the process and equipment to coat the beads or zeolites provides:
1. Distribution of the liquid coating formulation over the whole of the available bead/zeolite surface (e.g., ladling, spraying);
2. Continuous mixing of the bead/zeolite load in order to achieve an evenly coated product (e.g., rotation);
3. Continuous drying to solidify the film quickly (e.g., hot air); and
4. Removal of solvent vapor (e.g., plus dust generated, plus used drying air and atomizing air).

The coating system used and configuration of the entire coating system is dependent on the liquid sterilant/polymer matrix used, viscosity of said mixture, volatility of the components, and other considerations. Both systems are, for purposes of the embodiments, interchangeable and someone skilled in the art would understand the necessary process parameters at which to operate the system.

### EXAMPLE L - METHOD FOR COATING BEADS WITH HYDROGEN PEROXIDE

In the first example, a mixture of 50/50 W/V polyethylene glycol 3350 (Sigma Aldrich, St. Louis, Mo) / 30% hydrogen peroxide (Sigma Aldrich, St. Louis, MO) can be created. This mixture is stirred at 300 RPM on a heated stir plate at 30⁰C for 30 minutes. The mixture is then transferred to the holding tank for the feeding of the solution to the spray gun.

Titanium beads are loaded into the spray drum and heated via the heated air blower to a temperature of 30⁰C while being rotated for 20 minutes. After the beads have been sufficiently warmed, the coating process is started. During coating the beads move through an application zone in which a portion of the beads/zeolites receive some coating. Most of the time beads are in drying mode moving away from the application zone and recycled repeatedly through the application zone. In the coating operation (continuous) an equilibrium is maintained between coating composition application rate and the rate of evaporation of the solvent. Deviation from this equilibrium results in serious coating problems. These parameters are;
- spray gun position: 6-18 inches from the bed, 45⁰ angle to the beads bed. Not overlapping fan width nor too far.
- under loading the pan will result in situation where the beads do not cover fully the exhaust plenum, and the majority of the drying air stream will bypass the bead bed and drying efficiency will be low.
- air volume and temperature: drying is controlled by the quantity and temperature of the drying hot air, and the quantity of exhaust air. It is important to balance the inlet and exhaust air flow rates such that there is slight negative pressure in the chamber.

Also, the temperature difference between the inlet and exhaust must be within 20-30°C. It is important to monitor the following three temperatures:
1. inlet air temperature
2. tablet bed temperature
3. exhaust air temperature

In this system liquid is pumped through an orifice of 0.045 inch at relatively low pressure of 35 psig. Low pressure of 55 psig at the atomizer tip can be used. The beads are sprayed with the mixture set at 30⁰C and drying heat at 30°C for 20 minutes creating a coating of about 200 microns evenly distributed over the surface of all the beads.

The methods disclosed herein include one or more actions for achieving the described method. The method and/or actions can be interchanged with one another without departing from the scope of the claims. In other words, unless a specific order of actions is specified, the order and/or use of specific actions can be modified without departing from the scope of the claims.

The various operations of methods and functions of certain system components described above can be performed by any suitable means capable of performing the corresponding functions. These means can be implemented, in whole or in part, in hardware. Thus, they can include one or more Application Specific Integrated Circuits (ASICs) adapted to perform a subset of the applicable functions in hardware. Alternatively, the functions can be performed by one or more other processing units (or cores), on one or more integrated circuits (ICs). In other embodiments, other types of integrated circuits can be used (e.g., Structured/Platform ASICs, Field Programmable Gate Arrays (FPGAs), and other Semi-Custom ICs), which can be programmed. Each can also be implemented, in whole or in part, with instructions embodied in a computer-readable medium, formatted to be executed by one or more general or application-specific controllers. Embodiments can also be configured to support plug-and-play functionality (e.g., through the Digital Living Network Alliance (DLNA) standard), wireless networking (e.g., through the 802.11 standard), etc.

The steps of a method or algorithm or other functionality described in connection with the present disclosure, can be embodied directly in hardware, in a software module executed by a processor, or in a combination of the two. A software module can reside in any form of tangible storage medium. Some examples of storage media that can be used include random access memory (RAM), read only memory (ROM), flash memory, EPROM memory, EEPROM memory, registers, a hard disk, a removable disk, a CD-ROM and so forth. A storage medium can be coupled to a processor such that the processor can read information from, and write information to the storage medium. In the alternative, the storage medium can be integral to the processor.

A software module can be a single instruction, or many instructions, and can be distributed over several different code segments, among different programs, and across multiple storage media. Thus, a computer program product can perform operations presented herein. For example, such a computer program product can be a computer-readable, tangible medium having instructions tangibly stored (and/or encoded) thereon, the instructions being executable by one or more processors to perform the operations described herein. The computer program product can include packaging material. Software or instructions can also be transmitted over a transmission medium. For example, software can be transmitted from a website, server, or other remote source using a transmission medium such as a coaxial cable, fiber optic cable, twisted pair, digital subscriber line (DSL), or wireless technology such as infrared, radio, or microwave.

Features implementing functions can also be physically located at various positions, including being distributed such that portions of functions are implemented at different physical locations. Also, as used herein, including in the claims, "or" as used in a list of items prefaced by "at least one of" indicates a disjunctive list such that, for example, a list of "at least one of A, B, or C" means A or B or C or AB or AC or BC or ABC (i.e., A and B and C). Further, the term "exemplary" does not mean that the described example is preferred or better than other examples.

Various changes, substitutions, and alterations to the techniques described herein can be made without departing from the technology of the teachings as defined by the appended claims.

## Claims

1. A sterilizing system for sterilizing medical devices, the system comprising:
a first vacuum chamber sized to receive a medical device;
a conductive thermal assembly configured to at least partially surround the medical device when the medical device is disposed within the first vacuum chamber;
a first heater configured to provide thermal energy to the conductive thermal assembly, wherein the thermal conduction assembly conducts at least a portion of the thermal energy to the medical device;
a sterilizing subassembly including:
a solid-form sterilant having a liquid sterilant encapsulated within a matrix material; and
a second heater configured to provide thermal energy to the solid-form sterilant; and
a first depressurization subsystem configured, when the medical device is in the vacuum chamber, to produce a negative pressure environment within the first vacuum chamber, wherein the liquid sterilant within the solid-form sterilant off-gasses to generate gasified sterilant in response to the negative pressure environment, wherein the gasified sterilant expands into the first vacuum chamber;
a monitoring subsystem having a temperature sensor within the chamber configured to monitor an internal temperature of the chamber and modify heat delivered to the conductive thermal assembly such that the delivered thermal energy is at least sufficient to replenish latent heat of vaporization lost from producing the negative pressure environment.

2. The system of Claim 1, wherein the thermal conduction assembly comprises:
a plurality of thermally conductive beads that are configured, when the medical device is in the chamber, to at least partially conform to an external shape of the medical device.

3. The system of Claim 2, wherein the first heater comprises a resistive electrical heater in physical contact with at least a portion of the plurality of thermally conductive beads.

4. The system of Claim 1, wherein the sterilizing subassembly is disposed within the first vacuum chamber.

5. The system of Claim 4, wherein the first heater is configured to heat the conductive thermal assembly to a first temperature and the second heater is configured to heat the solid-form sterilant to a second temperature, wherein the first temperature and the second temperature are different.

6. The system of Claim 1, further comprising:
a second vacuum chamber in selective fluid communication with the first vacuum chamber, wherein the sterilizing subassembly is disposed within the second vacuum chamber.

7. The system of Claim 6, further comprising:
a valve in a fluid path between the first vacuum chamber and the second vacuum chamber, wherein the valve is configured to:
open provide fluid communication between the first vacuum chamber and the second vacuum chamber; and
close to fluidly isolate the first vacuum chamber and the second vacuum chamber.

8. The system of Claim 7, further comprising:
a second depressurization subsystem configured to produce a negative pressure environment within the second vacuum chamber, when the first vacuum chamber and the second vacuum chamber are isolated.

9. The system of Claim 7, further comprising:
a controller configured operatively connected to the first depressurization system and the first valve, wherein the controller is configured to:
operate at least the first depressurization system to produce the negative pressure environment within the first vacuum chamber and produce a negative pressure environment within the second vacuum chamber; and
open the valve to permit the gasified sterilant in the second vacuum chamber to expand into the first vacuum chamber.

10. The system of Claim 9, wherein the controller is further configured to:
re-operate at least the first depressurization system to reproduce the negative pressure environment within the first vacuum chamber and reproduce the negative pressure environment within the second vacuum chamber; and
reopen the valve to permit gasified sterilant in the second vacuum chamber to expand into the first vacuum chamber.

11. The system of Claim 9, wherein the controller is further configured to:
vent the first vacuum chamber after a predetermined time to establish atmospheric pressure within the first vacuum chamber.

12. The system of claim 1, wherein the solid-form sterilant comprises:
a liquid sterilant disposed within a polymeric matrix.

13. The system of claim 12, wherein the liquid sterilant comprises at least one of:
hydrogen peroxide;
peracetic acid; and
Performic acid.

14. The system of claim 1, wherein the solid form sterilant is disposed within a gas permeable packet.

15. The system of claim 1, wherein the depressurization subsystem comprises a vacuum pump in substantially sealed fluid communication with the first vacuum chamber.

## Patentansprüche

1. Sterilisationssystem zum Sterilisieren von medizinischen Vorrichtungen, wobei das System Folgendes umfasst:
eine erste Unterdruckkammer, dimensioniert zum Aufnehmen einer medizinischen Vorrichtung;
eine wärmeleitende Anordnung, dazu ausgelegt, die medizinische Vorrichtung zumindest teilweise zu umgeben, wenn die medizinische Vorrichtung innerhalb der ersten Unterdruckkammer angeordnet ist;
einen ersten Heizer, ausgelegt zum Bereitstellen von Wärmeenergie zur wärmeleitenden Anordnung, wobei die wärmeleitende Anordnung zumindest einen Teil der Wärmeenergie zur medizinischen Vorrichtung leitet;
eine Sterilisationsteilanordnung, die Folgendes umfasst:
ein Sterilisationsmittel in fester Form, ein innerhalb eines Matrixmaterials gekapseltes flüssiges Sterilisationsmittel aufweisend; und
einen zweiten Heizer, ausgelegt zum Bereitstellen von Wärmeenergie für das Sterilisationsmittel in fester Form; und
ein erstes Druckentspannungsteilsystem, ausgelegt zum, wenn die medizinische Vorrichtung in der Unterdruckkammer ist, Produzieren einer negativen Druckumgebung innerhalb der ersten Unterdruckkammer, wobei das flüssige Sterilisationsmittel innerhalb des Sterilisationsmittels in fester Form ausgast, um in Reaktion auf die negative Druckumgebung ein vergastes Sterilisationsmittel zu erzeugen, wobei sich das vergaste Sterilisationsmittel in die erste Unterdruckkammer ausdehnt;
ein Überwachungsteilsystem, einen Temperatursensor innerhalb der Kammer aufweisend, ausgelegt zum Überwachen einer internen Temperatur der Kammer und Modifizieren von Wärme, die an die wärmeleitende Anordnung geliefert wird, sodass die gelieferte Wärmeenergie zumindest ausreichend ist, um die latente Verdampfungswärme zu ergänzen, die durch Produzieren der negativen Druckumgebung verloren geht.

2. System nach Anspruch 1, wobei die wärmeleitende Anordnung Folgendes umfasst:
mehrere wärmeleitende Perlen, die ausgelegt sind zum, wenn die medizinische Vorrichtung in der Kammer ist, zumindest teilweise Übereinstimmen mit einer äußeren Form der medizinischen Vorrichtung.

3. System nach Anspruch 2, wobei der erste Heizer einen resistiven elektrischen Heizer in physischem Kontakt mit zumindest einem Teil der mehreren wärmeleitenden Perlen umfasst.

4. System nach Anspruch 1, wobei die Sterilisationsteilanordnung innerhalb der ersten Unterdruckkammer angeordnet ist.

5. System nach Anspruch 4, wobei der erste Heizer ausgelegt ist zum Erhitzen der wärmeleitenden Anordnung auf eine erste Temperatur und wobei der zweite Heizer ausgelegt ist zum Erhitzen des Sterilisationsmittels in fester Form auf eine zweite Temperatur, wobei die erste Temperatur und die zweite Temperatur verschieden sind.

6. System nach Anspruch 1, das ferner Folgendes umfasst:
eine zweite Unterdruckkammer in gezielter Fluidverbindung mit der ersten Unterdruckkammer, wobei die Sterilisationsteilanordnung innerhalb der zweiten Unterdruckkammer angeordnet ist.

7. System nach Anspruch 6, das ferner Folgendes umfasst:
ein Ventil in einem Fluidpfad zwischen der ersten Unterdruckkammer und der zweiten Unterdruckkammer, wobei das Ventil ausgelegt ist zum:
Öffnen zum Bereitstellen von Fluidverbindung zwischen der ersten Unterdruckkammer und der zweiten Unterdruckkammer; und
Schließen zum fluidischen Isolieren der ersten Unterdruckkammer und der zweiten Unterdruckkammer.

8. System nach Anspruch 7, das ferner Folgendes umfasst:
ein zweites Druckentspannungsteilsystem, ausgelegt zum Produzieren einer negativen Druckumgebung innerhalb der zweiten Unterdruckkammer, wenn die erste Unterdruckkammer und die zweite Unterdruckkammer isoliert sind.

9. System nach Anspruch 7, das ferner Folgendes umfasst:
eine Steuerung, dazu ausgelegt, mit dem ersten Druckentspannungssystem und dem ersten Ventil wirkverbunden zu sein, wobei die Steuerung ausgelegt ist zum:
Betreiben von zumindest dem ersten Druckentspannungssystem zum Produzieren der negativen Druckumgebung innerhalb der ersten Unterdruckkammer und Produzieren einer negativen Druckumgebung innerhalb der zweiten Unterdruckkammer; und
Öffnen des Ventils, um dem vergasten Sterilisationsmittel in der zweiten Unterdruckkammer zu ermöglichen, sich in die erste Unterdruckkammer auszudehnen.

10. System nach Anspruch 9, wobei die Steuerung ferner ausgelegt ist zum:
erneuten Betreiben von zumindest dem ersten Druckentspannungssystem zum Reproduzieren der negativen Druckumgebung innerhalb der ersten Unterdruckkammer und Reproduzieren der negativen Druckumgebung innerhalb der zweiten Unterdruckkammer; und
erneuten Öffnen des Ventils, um dem vergasten Sterilisationsmittel in der zweiten Unterdruckkammer zu ermöglichen, sich in die erste Unterdruckkammer auszudehnen.

11. System nach Anspruch 9, wobei die Steuerung ferner ausgelegt ist zum:
Lüften der ersten Unterdruckkammer nach einer vorbestimmten Zeit zum Errichten von atmosphärischem Druck innerhalb der ersten Unterdruckkammer.

12. System nach Anspruch 1, wobei das Sterilisationsmittel in fester Form Folgendes umfasst:
ein flüssiges Sterilisationsmittel, angeordnet innerhalb einer Polymermatrix.

13. System nach Anspruch 12, wobei das flüssige Sterilisationsmittel zumindest eines aus Folgendem umfasst:
Wasserstoffperoxid;
Peressigsäure; und
Perameisensäure.

14. System nach Anspruch 1, wobei das Sterilisationsmittel in fester Form innerhalb eines gasdurchlässigen Pakets angeordnet ist.

15. System nach Anspruch 1, wobei das Druckentspannungsteilsystem eine Unterdruckpumpe in im Wesentlichen abgedichteter Fluidverbindung mit der ersten Unterdruckkammer umfasst.

## Revendications

1. Système de stérilisation pour stériliser des dispositifs médicaux, le système comprenant :
une première chambre sous vide dimensionnée pour recevoir un dispositif médical ;
un ensemble thermo-conducteur configuré pour au moins partiellement entourer le dispositif médical lorsque le dispositif médical est disposé à l'intérieur de la première chambre sous vide ;
un premier appareil de chauffage configuré pour fournir de l'énergie thermique à l'ensemble thermo-conducteur, dans lequel l'ensemble thermo-conducteur conduit au moins une partie de l'énergie thermique vers le dispositif médical ;
un sous-ensemble de stérilisation incluant :
un stérilisant sous forme solide ayant un stérilisant liquide encapsulé à l'intérieur d'un matériau de matrice ; et
un second appareil de chauffage configuré pour fournir de l'énergie thermique au stérilisant sous forme solide ; et
un premier sous-système de décompression configuré, lorsque le dispositif médical est dans la chambre sous vide, pour produire un environnement sous pression négative à l'intérieur de la première chambre sous vide, dans lequel le stérilisant liquide à l'intérieur du stérilisant sous forme solide se dégaze pour générer un stérilisant gazéifié en réponse à l'environnement sous pression négative, dans lequel le stérilisant gazéifié se détend dans la première chambre sous vide ;
un sous-système de surveillance ayant un capteur de température à l'intérieur de la chambre, configuré pour surveiller une température interne de la chambre et modifier la chaleur distribuée à l'ensemble thermo-conducteur de telle sorte que l'énergie thermique distribuée soit au moins suffisante pour effectuer le réapprovisionnement en chaleur latente de vaporisation perdue en raison de la production de l'environnement sous pression négative.

2. Système selon la revendication 1, dans lequel l'ensemble thermo-conducteur comprend :
une pluralité de billes thermo-conductrices qui sont configurées, lorsque le dispositif médical est dans la chambre, pour épouser au moins partiellement une forme externe du dispositif médical.

3. Système selon la revendication 2, dans lequel le premier appareil de chauffage comprend un appareil de chauffage électrique résistif en contact physique avec au moins une partie de la pluralité de billes thermo-conductrices.

4. Système selon la revendication 1, dans lequel le sous-ensemble de stérilisation est disposé à l'intérieur de la première chambre sous vide.

5. Système selon la revendication 4, dans lequel le premier appareil de chauffage est configuré pour chauffer l'ensemble thermo-conducteur jusqu'à une première température et le second appareil de chauffage est configuré pour chauffer le stérilisant sous forme solide jusqu'à une seconde température, dans lequel la première température et la seconde température sont différentes.

6. Système selon la revendication 1, comprenant en outre :
une seconde chambre sous vide en communication fluidique sélective avec la première chambre sous vide, dans lequel le sous-ensemble de stérilisation est disposé à l'intérieur de la seconde chambre sous vide.

7. Système selon la revendication 6, comprenant en outre :
une soupape dans un chemin fluidique entre la première chambre sous vide et la seconde chambre sous vide, dans lequel la soupape est configurée pour :
s'ouvrir pour fournir une communication fluidique entre la première chambre sous vide et la seconde chambre sous vide ; et
se fermer pour isoler fluidiquement la première chambre sous vide et la seconde chambre sous vide.

8. Système selon la revendication 7, comprenant en outre :
un second sous-système de décompression configuré pour produire un environnement sous pression négative à l'intérieur de la seconde chambre sous vide, lorsque la première chambre sous vide et la seconde chambre sous vide sont isolées.

9. Système selon la revendication 7, comprenant en outre :
un appareil de commande configuré de façon fonctionnellement connectée au premier système de décompression et à la première soupape, dans lequel l'appareil de commande est configuré pour :
faire fonctionner au moins le premier système de décompression pour produire l'environnement sous pression négative à l'intérieur de la première chambre sous vide et produire un environnement sous pression négative à l'intérieur de la seconde chambre sous vide ; et
ouvrir la soupape pour permettre au stérilisant gazéifié dans la seconde chambre sous vide de se détendre dans la première chambre sous vide.

10. Système selon la revendication 9, dans lequel l'appareil de commande est en outre configuré pour :
refaire fonctionner au moins le premier système de décompression pour reproduire l'environnement sous pression négative à l'intérieur de la première chambre sous vide et reproduire l'environnement sous pression négative à l'intérieur de la seconde chambre sous vide ; et
rouvrir la soupape pour permettre à un stérilisant gazéifié dans la seconde chambre sous vide de se détendre dans la première chambre sous vide.

11. Système selon la revendication 9, dans lequel l'appareil de commande est en outre configuré pour :
mettre à l'air libre la première chambre sous vide après un temps prédéterminé pour établir une pression atmosphérique à l'intérieur de la première chambre sous vide.

12. Système selon la revendication 1, dans lequel le stérilisant sous forme solide comprend :
un stérilisant liquide disposé à l'intérieur d'une matrice polymérique.

13. Système selon la revendication 12, dans lequel le stérilisant liquide comprend au moins un de :
du peroxyde d'hydrogène ;
de l'acide peracétique ; et
de l'acide performique.

14. Système selon la revendication 1, dans lequel le stérilisant sous forme solide est disposé à l'intérieur d'un paquet perméable aux gaz.

15. Système selon la revendication 1, dans lequel le sous-système de décompression comprend une pompe à vide en communication fluidique sensiblement étanche avec la première chambre sous vide.
